(19) Europäisches Patentamt
European Patent Office
Office européen des brevets

(11) **EP 2 729 048 B1**

(12) **EUROPEAN PATENT SPECIFICATION**

(45) Date of publication and mention
of the grant of the patent:
**20.09.2023 Bulletin 2023/38**

(21) Application number: **12784318.3**

(22) Date of filing: **09.07.2012**

(51) International Patent Classification (IPC):
***A61B 1/24*** *(2006.01)*    ***A61B 1/00*** *(2006.01)*
***A61B 5/107*** *(2006.01)*    ***H04N 13/243*** *(2018.01)*

(52) Cooperative Patent Classification (CPC):
**A61B 1/000094; A61B 1/00158; A61B 1/00194;**
**A61B 1/24; A61B 5/065; A61B 5/1076;**
**A61B 5/1077; A61C 9/004; H04N 13/243;**
A61B 1/00016; A61B 1/00018; A61B 1/00172;
A61B 1/00174; A61B 1/00188; A61B 1/0019;

(Cont.)

(86) International application number:
**PCT/IB2012/001777**

(87) International publication number:
**WO 2013/008097 (17.01.2013 Gazette 2013/03)**

(54) **THREE-DIMENSIONAL MEASURING DEVICE USED IN THE DENTAL FIELD**

DREIDIMENSIONALE MESSVORRICHTUNG FÜR DEN DENTALBEREICH

DISPOSITIF DE MESURE EN TROIS DIMENSIONS UTILISÉ DANS LE DOMAINE DENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.07.2011 FR 1156201**

(43) Date of publication of application:
**14.05.2014 Bulletin 2014/20**

(73) Proprietors:
• **Duret, François**
**11560 Fleury d'Aude (FR)**
• **Querbes, Olivier**
**31520 Ramonville Saint-Agne (FR)**
• **Querbes-Duret, Véronique**
**31520 Ramonville Saint-Agne (FR)**

(72) Inventors:
• **Duret, François**
**11560 Fleury d'Aude (FR)**
• **Querbes, Olivier**
**31520 Ramonville Saint-Agne (FR)**

• **Querbes-Duret, Véronique**
**31520 Ramonville Saint-Agne (FR)**

(74) Representative: **Rhein, Alain**
**Cabinet BREV & SUD**
**55 Avenue Clément Ader**
**34170 Castelnau-le-Lez (FR)**

(56) References cited:
| | |
|---|---|
| **EP-A1- 1 600 804** | **EP-A1- 2 213 223** |
| **WO-A1-98/11815** | **DE-A1- 19 636 354** |
| **DE-A1-102008 031 054** | **DE-A1-102009 001 086** |
| **DE-A1-102009 026 248** | **US-A1- 2008 038 688** |
| **US-A1- 2009 227 875** | **US-A1- 2010 106 275** |
| **US-A1- 2010 165 080** | |

EP 2 729 048 B1

(52) Cooperative Patent Classification (CPC): (Cont.)
A61B 1/00193; A61B 1/05; A61B 1/0684;
A61C 9/0053

**Description**

The present invention relates to a new secure three-dimensional measuring device through contactless high-precision and wide-field optical color impression without structured active light projection, especially for dentistry.

The present invention ensures the structural integrity of the human body and an accuracy in the range of one micron. It is applicable namely in the medical and dental fields for intra-oral picture recordings and assistance in diagnosis.

It may include:

1) a miniaturized three-dimensional reading system using no active or structured light projection for measuring the dimensions of the object, consisting of

a) one or more CCD- or CMOS-type electronic sensors and its associated optical system,
b) eventually one LED or OLED lighting of one or several wavelengths permitting to diagnose eventual pathologies at the surface of the teeth or the gums,
c) one or more accelerometers/gyros/3D-magnetometers for assisting, limiting, even replacing one or several sensors.

2) a central unit for converting analogue/digital data and management data,
3) associated software permitting 3D spatial analysis almost in real time, temporal analysis for analyzing the movements of the measured objects, colorimetric analysis for analyzing the color of these objects in direct correlation and in real time with the surfaces measured in 3D providing assistance for the diagnosis through reflection, global or selective penetration of the carefully selected LED/OLED light radiation,
4) an IRM communication "hardware" and "software" set (screen, keyboard, modem ...).

This invention permits to solve the fundamental problems the systems for recording optical 3D impressions are facing. It provides real-color and real-time information for the dentistry. It measures the object without projecting any structured active light with an accuracy of at least 10-15 $\mu$m at a field depth of at least 15 mm and a surface of at least 20 x 30 mm on the teeth located within 10 mm of the front lens of the camera.

There exist a large variety of methods for recording optical impressions in the mouth or on a model for making prostheses or a diagnosis. By the term "optical impression" first introduced in 1973 by the inventor of this technology, Francois Duret, in his thesis for the second cycle (DDS) under the title "Optical Impression" No. 273, the 3D measuring and diagnostic analysis of the oral and medical environment by contactless optical means, in substitution of the traditional impression methods with paste or probing.

In the dental field the works by Dr. Duret, described i.a. in a number of articles and in his patents dd. May 9, 1980 (FR 80.10967 or US 4,663,720 and 4,742,464), April 14, 1982 (BE 0,091,876 - US 4,611,288), November 30, 1982 (EP 0110797, US 5,092,022), March 27, 1984 (FR 84.05173), February 13, 1987 (FR 87.02339 or US 4,952,149) or also June 26, 1992 (FR 92.08128 or PCT WO 94/00074) have been echoed by many authors since the early 1980s, as we will see in the various technologies, which can be summarized as follows.

1) The techniques using the projection of active or structured light.

The simplest method used by these systems consists in projecting on the object structured light, which may be a dot, a line, even a full grid. This light will scan the object and is followed by one or several CCD or CMOS 2D cameras positioned at an angle ranging between 3° and 10° with respect to the axis of the light projection. These techniques have been widely known for several decades and are very well described in the article by G Hausler and Col. « light sectioning with large depth and high resolution » in Appl. Opt. 27 (1988). They have been the object of numerous developments and are used in particular by the desktop scanners in dental laboratories.

A more sophisticated method consists in projecting onto the teeth a structured active light in the form of a varying-pitch grid. The most common technique for this kind of fringe projection has been described for the first time by M. Altschuler and Col., under the title "Numerical stereo camera" SPIE vol 283 3-D (1981) Machine perception, which publication has been echoed by other authors such as M Halioua and Col. « Automated phase measuring profilometry of 3D diffuse objects » in Appl.Opt. 23 (1984). It consists in projecting a series of varying-pitch grids. The grid with the wider pitch serves for providing general information and the global position of the lines in z, the finest line for refining the accuracy of reading.

All these works and inventions have led to many embodiments and to more than twenty commercially available systems (F.Duret, the dental floss No. 63, May 2011, "the great adventure of CADCAM at IDS in Cologne" 14-26). We will cite for example the systems using a spot scanning system (Cera from Cera system, GN1 from GC and Nikon), a line scanning system (Titan from DCS, Ekton from Straumann), a varying-pitch frame scanning system (Cercom from Degudent, Digident from Hint-Els, Everest from Kavo, Lavascan from 3M, Zeno from Wielan or Wol-ceram from Wol-dent) .

These systems cannot be used in the mouth because they are too slow (1s to 1mn). The slightest movement by the patient or the operator impedes the full reading and the necessary correlation of pictures for transforming a 2D cross-sectional display into a 3D image. Furthermore, there is no information between the lines, which requires a series of readings in different directions, which further increases the reading time significantly (up to 4 minutes per tooth for the complete readings) .

Finally, more recently, in order to more easily determine the spatial position of the projected fringes, the chromatic profilometry technique has been provided, which uses the varying-color fringes. It has been described as profilometry by Cohen Sabban, BV F 2758076 and is the object of a marketing under the name Pro50 (Cynovad - Canada).

In order to meet the intra-oral reading requirements, faster systems has been provided. The first one has been marketed in France in 1985 under the name of Duret system (Vienne - France) and used the system of profilometric phase in conical projection as described in the patents (FR 82.06707 or US 4,611,288), (FR 82.20349 or US 5,092,022} and (FR 87.02339 or US 4,952,149). This technique has been adopted with great success by Moermann and Brandestini in their Patents 4,575,805 and 4,837,732 or in their books dealing with the issue as "Die Cerec Computer Reconstruction" in 1989, "CAD/CIM in Aesthetic Dentistry" in 1996 or also "State of the art of CAD/CAM restoration" in 2006. This method has been improved gradually as we can see in the patent by Jones, T.N. of 1999 (U.S. 6.409.504).

This is an active and structured light projection technique in the form of a frame projected onto the teeth according to parallel or conical radiation with a slight phase shift (generally n/2) and performing a series of 2D picture acquisitions (in 100 ms), the third dimension can be found provided the patient and the camera are perfectly still while recording the successive pictures, which remains difficult during a clinical action, the more since the electro-optical organs of the camera are mobile.

Other slightly different systems, but which use structured active projection in the mouth, have been provided: The simplest one is the "OralMetrix", which consists in projecting one single type of grid onto the surface of the teeth, as described in FR 84.05173). This is therefore an active triangulation associated with one single projection of structured light. One single camera reads the deformation of the grid and, by comparison with a stored grid, derives the distance z from it, the acquisition of six pictures per second associated with a 2D view of a deformed grid makes the system inaccurate and unstable during the picture recording.

The second system is the "directScan" from the company Hint-Els (USA). It combines the fringe projection and the phase correlation. This method takes place in two steps: projection of two series of orthogonal grids with different pitches, one after the other, then correlation of the pictures obtained depending of the position of the dots at the level of the pixels of the CCDs. This is an improvement of the profilometric phase, but the processing time is about 200 ms, which makes its use very difficult in the mouth. The measures are often erroneous.

The third system provided is the iTeo system from de company Cadent (US.0109559) based on the principle of the "parallel confocal image" where many 50 $\mu$m laser dots are projected at different field depths. This scanning of the target area has the advantage of having one single axis of image recording and rerecording of images, but takes about 300 ms. The apparatus must therefore not move during the recording of images. In addition, since this technology is complex, the iTero system is particularly voluminous, which limits the recording of images in the depth of the mouth.

The fourth system has been provided by G. Hausler (US 2010.0303341). Several structured light grids of different orientations are projected onto the arch. This permits to find the third dimension immediately through correlation between the first deformed grid and the next ones. This method permits to record only one image, but has the disadvantage of being capable of measuring only the dots of the deformed grid and not all the dots of the object itself.

In these methods based on active and structured light projection, we obtain several 2D images permitting to reconstruct the analyzed object in 3D. These methods are the more accurate as the projected light is fine and calibrated and as the moving organs are stable over time. Unfortunately, none of them measures the object itself, but only the deformation of the projected light, which limits the number of measured dots and can hide important areas for the exact reconstruction of the analyzed 3D surface.

Furthermore, it very often requires the object to be coated with a white layer referred to as coating, or to use special plasters when a model is measured. Indeed, the specular reflection of the teeth is very sensitive and responds in a varying way to the structured light projected depending on its own color.

This also has a major drawback as regards the accuracy of the measurement. The structured active light, because of its power, penetrates into the surface layers of the tooth, adding inaccuracy to the exact determination of the outer surface.

The calibration of these devices is complex and the mounting is always very complex and expensive.

Finally, since the angle of projection is often different from the angle of recovery of the image, the shadow effects can lead to the presence of uncoded shadow areas, which requires many manipulations. It should also be noted that we have no information between the lines.

Some systems have tried to limit the projection of structured light without removing it. To this end, they have associated a very small projected portion with a conventional 2D stereoscopic vision. One uses two identical cameras and projects a line or a target having a varying shape onto the object and moves the whole while scanning the surface of the object. The two 2D cameras form a conventional stereoscopic unit, both information of which are correlated thanks to the projected target visible in the two pictures. This system is marketed by means of the T-scan 3 sensor from Steinbichler Opt. (Neubeuern - Germany) or by Uneo (Toulouse - France). These methods, which have the same drawbacks as the methods described above, could never be applied to dentistry, because they in addition lack precision and, in particular, they require the projected target to always be displayed, which remains difficult on highly specular or uniform surfaces as in the case of the teeth.

2) The techniques, which do not use active or structured light projection.

The first proposal to use a stereoscopic intra-oral system was made by D. Rekow (J. of Dent.Practice Administration ; 4 (2) 52-55 (1984). In this system, it is necessary to make several acquisitions, with a reference fixed on the teeth, then to read these frames by means of a Kodak Rikonix device. This ancestral method, well known under the name of stereoscopic, has proved inaccurate and time-consuming for its implementation. This method was recently proposed again by Denzen Cao US 2009.0227875 (Sandy - USA) and by Steinbichler Opt. EP 2,166,303 (Neubeuern - Germany) without any improvement over the system by Rekow, in particular the resolution of the field depth, the determination of the reference dots and the accuracy, which is a crucial problem during the recording of intra-oral pictures corresponding to a close stereoscopic, has not been addressed. Such a system cannot be carried out in the mouth if we want to achieve an accuracy of 20 $\mu$m at a field depth of 20 mm with the object placed within 5 mm of the front lens.

The same remarks can be made for the systems using the technique referred to as "3D from motion" described, for example by C. Tomasi and Col. « Shape and motion from image streams under Orthography ; a factorization Method » dans Int. J. of Computer Vision 9 (2) 1992. This system no longer uses active light, as seen before, but only a passive illumination of the area measured by a conventional stereoscopic vision with two cameras having the same resolution. Unfortunately, under conventional circumstances as described by the authors, the correlations of pictures without projected target and the abundance of areas without coding make the use of this system impossible on the teeth. It does not solve the problems evoked by Rekow. This is the reason why recently the system by Active Wavefront Sempling (AWS), based on the Biris system, marketed by 3M with his Lava Cos camera has been introduced on the market in 2008 (Rohaly and Co. US Patent 7,372,642). This system uses a single view scanning, thanks to a rotatory disk, a very small portion of the object. The diameter of the position of the view in the focal plane and the mechanical variation of the focal length with respect to the optical axis of the mounting permits to know the spatial position of the small area measured at a small-field depth. Unfortunately, the system is complex and expensive for its implementation and the very small scanning area requires the operator to slowly move over all the areas to be measured.

Another device is disclosed in document US 2008/038688 A1. In this device, the resolution of the scanned data for the ancillary parts may be less than for a target part.

Similarly, document US 2010/106275 A1 discloses a scanner device being controlled to apply a first scanning resolution for a first portion of a physical impression and a second scanning resolution for a second portion of the physical impression.

Whether they are laboratory systems or intra-oral cameras, including the one we developed, all these systems do not provide the required qualities to have a quality information in order to make prostheses or diagnoses. A more thorough analysis shows that these cameras have several very important drawbacks, in the very principle of the methods used. These drawbacks are unavoidable, because they are related to the choice of these methods.

a) All these systems, whether in the mouth, on the skin or in the laboratory (on model) use the surface scanning by mechanical, optical or electro-optical means. Although this scanning of fringes or frames is very fast, the fact remains that it requires a movement in the camera itself, which movement can cause blurry areas or parasitic movements, which often lead to the rejection of part of the pictures.

b) This scanning significantly limits the already considerably reduced field depth in a macroscopic picture (of a few cubic centimeters).

c) the dots of the surface of the object are not measured, but the deformation of a light projection on the surface of this object is measured. This first feature requires developers to cover the teeth with a white layer referred to as "coating", which degrades, in principle, the actual measurement of the object. This is in fact often expressed both as inaccuracy and inconvenience in the use of cameras in the mouth (Beuttell, J Int.J.Cumputerized Dent. 1998 1:35-39).

Besides, this layer is often mandatory if we do not want to have any penetration, thus inaccuracy, in measuring the exact position of the tooth surface, crystalline organ per excellence where a sufficient signal-to-noise ratio is required.

d) This has led some manufacturers to use radiation, making the tooth "opaque" as do the blue or UV rays. This is why the present inventor proposed in 1985, presented to the ADF, the use of an argon laser. This can be restrictive for the user, even dangerous, for the patient.

e) even more, not measuring the object, but the deformation of the projected light, either a dot, a line, a frame of a varying shape or a phase of this light, removes all possibilities of having a perfect match in real time between the color, the color shade of the object and its measurement. The only color that we can have in real time is the color of the projected light,

f) There is no immediate solution allowing the clinician to continue his surgical procedure if a component fails, which is crucial during a clinical procedure.

g) the transition from 3D reading to 2D color reading, when it is used for diagnosis, is completely impossible in dentistry, because we will recover only a monochromatic image representing the light of the fringes.

h) finally, the techniques of analysis by profilometry or scanning require recording multiple pictures of the same spot in order to be able to extract the third dimension. This results into a risk of distortion of the data between the first picture and the last pictures, leading to large errors in correlation and accuracy. The "movement" has always been an enemy of this type of technology.

Finally, if it is possible to measure a tooth, in most cases a measurement of the projected light is carried out and not a measurement of the object itself. In the case in which we do not use projected light, we must use complex and expensive defocussing systems. This explains why the proposed cost is particularly high. As for the only stereoscopic systems that have been provided for decades, they have nothing innovative and are therefore inaccurate, time-consuming to handle, complex and very expensive to be implemented.

No simple and above all secure solution has been found to meet the tooth/camera proximity, fast carrying out, required accuracy, the measurement of the actual color and field depth on a quite large surface.

The object of the present invention is to solve the aforementioned drawbacks by providing a new and very secure stereoscopic method for intra-oral reading combining a very fast, even instantaneous dynamic 3D reading, a measuring at a field depth corresponding to the intended application and the availability almost in real time of a real 3D or 2D color display, all this leading to a very accurate digitalizing, a data storage and transfer without using structured active light or addition of a "coating" covering the teeth.

The three-dimensional measuring device used in the dental field according to the invention is aimed at measuring in the absence of active or structured light projection, it comprises means for capturing images as well as data-processing means for said images, and it is characterized in that said image-capturing means are comprised of means designed capable of permitting to simultaneously, or nearly simultaneously, capture at least two images, one of which is fully or partially included in the other one, said included image describing a field that is narrower than that of the other one, and its accuracy is greater than that of the other one.

This invention solves the problems set forth by providing an adaptable, inexpensive solution usable in all dental and medical offices, but also as hand-held instrument in dental-prosthesis laboratories, in a simplified and patient-friendly form.

In particular, it solves the many problems mentioned above:

1) Through a new and original organization of the traditional dental stereoscopy, we limit the problem of the blind spots between the two picture recordings corresponding to the difference between the optical axes, which is crucial for an object close to the front lenses of the mounting, as teeth, in the mouth always are.

2) By using an original software arrangement, in case of failure of one of the sensors during the clinical procedure, it is possible to obtain a stereoscopic picture by means of one single sensor, which solution is simple, inexpensive and little bulky in the mouth.

3) By eventually adding a 3D accelerometer/gyroscope/ magnetometer, it is possible to accelerate and facilitate the correlation of the pictures with each other, especially in the event of failure of one of the sensors.

4) By choosing different focal lines, it is possible to solve the problems of accuracy and speed of clinical optical recording of an impression in the mouth. This also permits to combine or separate a general, less accurate recording on a wide field and a fast and accurate recording on a narrower field depending on the clinical need.

5) By choosing new lenses, in particular the liquid lenses, it is possible to eliminate the complex mechanical adjusting equipment, which ensures a measuring at an effective field depth in dentistry on objects very close to the measuring system because of the very small intra-oral space.

6) By not using measurements of deformation of structured active light, we work directly on the actual surface and in color of the body images. This permits for example to manually or automatically select certain parts of the human body, for example to identify the teeth and gums separately.
This also permits:

- Not to be compelled to cover the measured object with the "coating", which is unaccurate and tedious

- To have no penetration of measure-vector light inside the teeth, thanks to the abandonment of active structured light projection.

- To use the color of the read areas, in order to facilitate the matching of homologous dots, which is crucial in the mouth where the surfaces remain regular and uniform.

- To make highly effective and to reduce the reading time for measuring a complex surface (full arch) or the movements of these surfaces (upper arches with respect to lower arches).

- To enable self-calibration, eliminating any adjustment over time.

- To avoid any blur effect due to "movement" during the recording of pictures.

7) For the implemented means, the device is simple as to its manufacture, which makes it particularly resistant.

This also permits:

- to significantly reduce the manufacturing cost, hence the sale price, in particular from the democratization of the electronic components used, such as CCDs, CMOS or LEDs,

- to permit a reduced power supply, which can be provided by a USB-compatible connection with all types of computers or just a battery power-supply,

- to have CMOS or CCD sensors in a predetermined, immutable and fixed spatial position with respect to each other during manufacture, avoiding the need to know the movements of the object or cameras (with respect to each other), reducing the problem of disparity to a simple problem of density correlation in the scatter diagram.

- Being able to pass from a 3D image, spatial analysis, to a 2D image, planar analysis, useful for common diagnostics in dentistry without using software manipulations.

- To have the 3D display on standard 3D screens, which is not the case without complex processing of the present intra-oral systems.

The present invention relates to a new three-dimensional and temporal measuring device by means of optical color impressions in the mouth ensuring its structural integrity, namely applicable in the dental field for intra-oral recording of pictures, but also ensuring in these areas an assistance for dental diagnosis.
In accordance with the present "hardware" mounting there is provided a "software" method that meets the requirements of fastness and accuracy necessary for the specialist in dentistry and permitting to limit the stereoscopic vision to one or two sensors.
It is comprised of:
An miniaturized original stereoscopic system comprised of at least two sensors, of which:

1) one views a wide average-precision field and the other one a narrower field with higher accuracy fully or partially included in the previous field.

The wide field permitting a sufficiently large general recording of images in order to avoid a long and tedious scanning of the mouth for the practitioner.
since some areas are particularly strategic and require higher precision, a narrow field is included in the wide field, which permits to detect specific information where this is necessary, without being obliged to scan the entire mouth. This also permits to better define certain important homologous spots for the correlations between pictures.

It also permits the "software" to operate almost in real time, as this partial or full inclusion of the small field in the large field permits to very quickly find the position of the specific and highly localized area in a wider space.

It is obvious that these sensors can be multiplied when one wants to measure larger clinical areas, both at the level of the large field and at the level of the small field.

2) The optical systems associated with the sensors have different focal lengths, in order to permit two different levels of precision. The images received by the sensors, such as for example the CCDs or CMOS included in the head of the camera, are therefore a general image with an average accuracy, for example in the range of 20 pm and a complementary image with more information and a higher accuracy (5 to 10 $\mu$m) fully or partially included in the wide field. It is therefore unnecessary to scan the entire mouth to have accurate information required for by only less than 5% of the total area.

3) The advantage of this system is to facilitate the correlation of the two fields, since they are very similar, but also to limit the number of sensors without having to use clock or pulsed reading systems. Indeed, the approximation of the two fields shows that a single wide-field sensor or two sensors can be used without any complex electronic system. It also permits to avoid the use of light- or image-returning mirrors, which are always fragile and very voluminous in the mouth.

4) The fields are read by one or several electronic sensors, which can be of the color or monochromatic CMOS or CCD type generating the information necessary for calculating the color 3D or grayscale information. These sensors thus perform a measuring of the real-time color or black and white intensities. The measured color will thus be the actual color of the teeth and gums.

This is very important, because it permits i.a.:

  a. to automatically separate the teeth from the gums in the images.

  b. to identify some important colors for the CADCAM software

  c. to measure the color of the tooth on a three-dimensional surface.

5) This information is treated either by way of a video, in order to allow the operator and his assistants to follow in real time the movements of the camera in the mouth or, after an analog-to-digital conversion in a digital way that permits to have an almost real-time color 3D reconstruction and to be able of taking advantage of the dental CAD/CAM software processing, or a dual video and digital processing providing the operator with all the available information.

This will also allow the operator, as we will describe at the level of the "software", to know and come back to the areas that have been insufficiently measured in real time.

6) The optical system reading the scene has two different focal lengths. The advantage of this device is to be able to have:

  a. a focal length that does not require high precision, and to be able to have a unique fixed focal length without adjusting system. Tt is indeed optically possible to have a 20 x 30 x 15 mm field at 10 mm from the lens for an accuracy of 20-25 $\mu$.
  b. a high-precision focal length (5 to 10 $\mu$m), but the field depth of which is included in the previous one. The scanning in z will thus always be simple and known a priori. The scanning in z (field depth) will thus be limited to some 5 to 10 different levels.
  c. a high-precision focal length and variable zoom permitting to freely choose and increase the desired accuracy.

7) In order to facilitate the reading in the mouth by the practitioner, without any need of monitoring his screen, it is foreseen by the invention that the device includes means for projecting at least one circle of colored light surrounding the included image field, and/or the field of the other image:

  a. Eventually and preferably, the existence of a mark, for example a red circle, projected onto the scene in the picture indicating where the exact reading is located in the reading of the wide field.

  b. Eventually and preferably, the existence of a mark, such as a blue circle, projected onto the scene in the picture indicating where the edge of the wide field is located.

8) In order to avoid unpleasant and dangerous interruptions in the clinical reading in the mouth, a 3D accelerometer/ gyroscope/magnetometer is eventually and advantageously added, in order to facilitate the correlation of the pictures, even to compensate for a possible failure of one of the sensors. This device, placed in the vicinity of the sensors, provides general and continuous information on the spatial position of the camera.

This also permits, thanks to the "software" introduced, which is an inseparable part of the invention, to work with only one single sensor, the wide field or the narrow field, depending on the clinical needs, since some actions require a general study as in orthodontics, or a very accurate detection as for the localized unitary reconstitution.

9) While measuring on gypsum generally benefits of a good lighting, this is not true for readings in the mouth. Eventually and advantageously, the addition is provided of a passive and unstructured lighting by LEDs of one or several wavelengths permitting to measure specular or Lambertian smooth surfaces without deposition of coating on the surface of the mouth. Not using structured light also avoids the operator from turning off his professional lighting, which greatly facilitates his clinical work,

10) The information detected at the same time or with an extremely short shift avoids any movement causing redhibitory blur due to the movement of the operator or the patient.

11) In order to limit the blur phenomena, an anti-blur hardware system, or a "flash LED" system with a very fast pulse of the unstructured LED lighting or also a software that can be of type: anti-blur system in photographic cameras, is eventually added.

12) With the present invention is associated, for processing and displaying the data from the sensors:

a. a central management and analog/digital conversion unit without the slightest need for mechanical, optical or electro-optical scanning, structured-light projection permitting to calculate the 3 spatial dimensions and eventually the fourth dimension corresponding to the times of the movements of the measured objects.

b. original software permitting the use of a single sensor permitting a 3D detection almost in real time, in order to compensate for a possible failure of one of the sensors or to limit the volume of the camera.

c. a data transmission via cable, telephone or wireless.

d. a complementary processing, dialog/display with the operator, data transmission and storage hardware system.

An original software system including:

1) A real-time 3D reconstruction diagram starting from two 2D-image streams from both cameras,

2) A real-time 3D reconstruction diagram starting from a 2D-image stream from a single camera and an acceleration data flow from the accelerometer

3) An algorithm for finding dots of interest on the three algorithms for searching an optical trace (projection of the same 3D dot on several different cameras) by calculating dots of interest and matching through the images

4) An algorithm for real-time automatic sequencing of the stream of images into spatially coherent subsequences

5) An algorithm for estimating in parallel the camera positions in space and the coordinates of the 3D dots thanks to the optical traces

6) An algorithm for 3D interpolating the scatter diagram

7) An algorithm for polygonizing 3D scatter diagrams and calculating the texture

8) An algorithm for scaling the 3D reconstruction

9) Two algorithms for enhancing the spatial accuracy

Global organization of the algorithm;

The image stream proceeding from the cameras is processed in real time so as to produce a first 3D reconstruction displayable by the user as he moves the system in the vicinity of the object. The real-time 3D global reconstruction scheme and the organization of the data vary depending on the availability of the two cameras.

Each newly acquired picture is first of all processed by a algorithm for searching for an optical trace. Starting from the correspondences, a sequencing algorithm then updates the sequencing of the video stream for a better temporal performance. A parallel estimation algorithm can then permits, thanks to the optical traces

a) to find the positions of the cameras in the space at the time of acquisition

b) to generate the 3D scatter diagram projecting on the optical traces.

The generated scatter diagram is then interpolated, in order to obtain a denser diagram, and an implicit interpolation function is calculated. Thanks to this function, a textured polygonization of the surface to be reconstructed can be obtained. In this step, it is also possible to calculate quality indices of the final scatter diagram. Some of them or some areas can thus be labeled as invalid.

The textured surface is then displayed on the screen, eventually with adapted annotations to indicate the areas, which are still invalid.

The surface generated in real time is a representation without spatial dimension representing a scale factor near the reconstructed area. This scale factor is calculated by an algorithm when the acquisition is complete.

Finally, the final 3D model can have its accuracy enhanced by an algorithm, so as to have the most accurate possible reconstruction. This algorithm re-calculates a 3D scatter diagram taking into consideration all the acquired pictures. This diagram is then interpolated by the algorithm. Finally, an "space carving" algorithm reconstructs the global 3D model.

There is thus provided a device universal as to its field of application, meeting numerous requests in terms of cost, accuracy and diagnostic imaging in dentistry and medicine.

This system can for example be applied, in an evolutionary form, to any 3D acquisition requiring good accuracy including any human body surface, the acquisition of data related to the architecture and requiring high precision, or the industrial production processes. It is thus possible to scan the object measured with the single or multiple sensor, to move the object in front of the sensor(s) or to move both, sensor and object.

We remind that the elements permitting this measurement are made in real time and with a different accuracy, which permits to improve the reading of certain areas thanks to the narrow-field camera, while facilitating, thanks to the wide-field camera, a fast correlation with other captured images.

Other objects and advantages of the present invention will become clear from the following description, which refers to an embodiment of the method, given by way of an indicative and non-restgrictive example. The understanding of this description will be facilitated when referring to the attached drawings, in which:

- Figure 1a is an overall representation of the prototype made, including the camera, the connectors, the computer (here a laptop) and eventually a casing containing the processing cards.

- Figure 1b is a diagram showing the detail of the configuration of the invention.

- Figure 2 shows a view of the prototype made, highlighting the very small dimensions of the camera, thanks to the technique chosen and permitting its introduction into the mouth.

- Figure 3 shows a longitudinal cross-sectional view of the camera (1) including the image acquisition system (optical system and CCD or CMOS sensors) located in the head, in direct views (3a and 3b) .

- Figure 4 shows a frontal cross-sectional view of the head of the camera (1) according to the configuration we have just seen in drawings and 2 and denoting the covering of the wide and narrow reading area.

- Figure 5 shows the global volume analyzed by the wide-field camera and the small-field camera.

- Figure 6 shows the different levels of field depth provided by the use of variable focal length or the liquid lens analyzed by the wide-field camera and the small-field camera.

- Figure 7 shows the illustration of the pictures obtained by the wide-field camera and the small-field camera and 3D modeling obtained.

- Figures 8a, 8b and 8c show the automatic determination by software of the homologous dots on a plaster model (8a), in the mouth (8b) and the resulting scatter diagram (8c).

- Figures 9a and 9b represent the arrangement of the LEDs in passive lighting (9a) and the target projected onto the teeth (9b) permitting the practitioner to know the area scanned by the high-precision camera.

- Figures 10a, 10b and 10c represent a view obtained with white light (10a), blue light (10b) and composite blue and white light (10c).

- Figure 11 shows the aperture in the head of the camera permitting the jet of air, in order to remove saliva or blood and the protective heating glass avoiding the presence of moisture during the recording of an optical impression in the mouth.

- Figure 12 shows the general diagram of the software part, from the integration of the acquired images to the final 3D reconstruction to scale.

- Figures 13a, 13b and 13c represent three algorithms for using the acquired images in real time in the case in which two cameras are used simultaneously.

- Figure 14 shows the two possible reconstruction strategies when one single camera is used.

- Figure 15 shows an exemplary calculation of an optical trace by "tracking" of the dots of interest.

- Figure 16 shows the simplified steps of the algorithm for real-time 3D reconstruction.

- Figure 17 shows the organization of the algorithm for enhancing the accuracy.

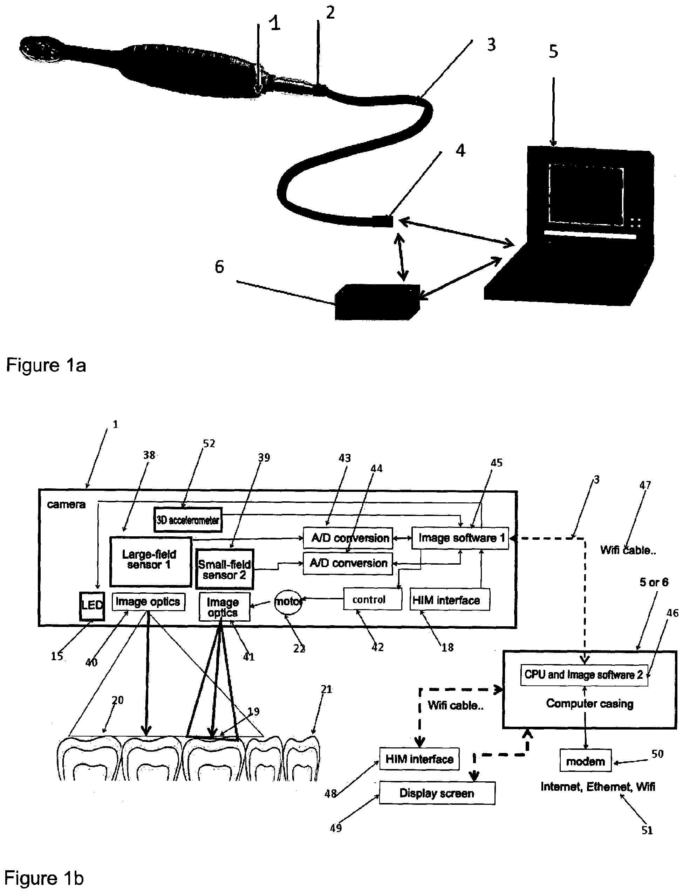

As shown in Figure 1, the present invention, presented in the form of a prototype, in the form of a schematic design photo in the following figures, relates to a measuring and/or diagnosis device that will find a particular interest in the fields of dentistry.

As shown in photo 1a, this device includes a camera with focal length (1) using the technology described in the invention, a connection (2) between the camera (1) and the cable (3) for supplying and transferring data, the connection (4) between the cable and the computer (5) being of the USB type and the casing (6), which can be placed in between for adding a driving card for the processor of the camera and/or processing the image if they are not placed in the camera or in the computer.

This same camera can use a wireless WiFi-type connection for transmitting images or data proceeding from the images, and a charger system for charging rechargeable batteries for the power to to supplied to the camera.

The electronic part, which can be entirely included in the body of the camera (9-12) or shared between the camera, the casing (6) and the computer (5). It includes an electronic system located behind or near the sensors, ensuring the management of the latter, but also of the LEDs illuminating the impression recording area. This electronic system also includes:

- a central management unit that can collect, store and order the data of the sensors in a language understandable by a universal PC. It will eventually also be capable of converting data having analog values into digital values if this function is not transferred to the remote PC. Not having to manage a system for projecting masks or fringes significantly reduces the central unit to its bare minimum: the management of a stereoscopic color picture camera.
- a LED control card, under the control of the central unit and/or software of the PC, capable of triggering preferably a particular LED depending on the programs being implemented. Indeed, the LEDs will be controlled alternately or together, or according to a varying order depending on the program being implemented. The function is in the form of a simple order, but it is good to mention it.
- a standard power-supply card capable of operating on USB or on battery power (e.g. AC/DC). Depending on whether we have a free system (without wire connection) or a wired system, the power supply will remain light, taking into consideration the low power consumption of the components being implemented. Our camera will thus be the first one that can have a wireless connection.
- eventually, a miniaturized memory card eventually included in the camera, permitting to store the pictures and to transfer them to the computer using a transportable medium without needing a USB connection or a wireless communication.

A standard laptop (5), netbook or desktop PC containing the management and program and data processing software can be added to the unit when everything is not included in the camera or/ and the intermediate casing (6). It is capable of reproducing the information in a 2D or 3D form visible on the screen, but also to send the measures to more or less remote centers (internet, Wifi, Ethernet ...) in a standard form similar to any CAD/CAM system (STL..) or in a specific form, by means of language translation software. In this computer, before having a miniaturized computing unit, will be installed the 3D restitution and camera control software.

Thus, the connection between the camera and the computer can be wired or wireless.

According to an embodiment of the invention, the wireline connection (3) is preferably via a self-powered USB connection (4) with a specific port (2) at the side of the camera (1). This specific connection (2) is designed so that it is adaptable to any camera shape and design.

Likewise, and according to an embodiment of the invention, the connection can be wireless, for example in Wifi mode, and this is not restrictive. In this case, the antenna will be included in the camera or connected instead of the specific connection (2). Likewise, on the computer (5) or the intermediate casing (6), an antenna for sending and receiving data corresponding to the commands given by the program located in the camera, in the computer (5) or the intermediate casing (6) will be inserted into the USB connection. This arrangement will permit fast, friendly and easy communication, irrespective of the configurations of the medical, dental offices or dental prosthesis laboratories.

In the same way and still according to an embodiment of the invention, the unit formed by the processing cards, the CPU and the display will be installed in the intermediate casing (6) so that the unit according to the embodiment can be integrated into a professional piece of furniture, such as the unit of the dentists or the work-bench of the dental technicians.

According to an embodiment of the invention, the computer (5) will be of a standard type with an incorporated or separate screen, such as a PC or the like (Mac ...). This computer will use standard cards specifically programmed for controlling the camera or specific control cards, which will be placed on the bus.

In the event the computer could not be equipped or when it is previously present in the dental-care unit, an intermediate casing (6) will be positioned between the camera and the computer in order to compensate for this lack. Similarly and for the same function, this casing will be positioned downstream of the computer and the USB connection (4) of the connection will be connected directly to the USB port of the computer, without any intermediate part. This will generate a specific language that can be interpreted by each CAD or CAM application used in the professional workplace.

Figure 1b shows the detail of the configuration of the invention. This diagram is comprised of two major entities, the camera (1) and the computer (5), which may be substituted with a specific and dedicated casing (6).

After having chosen a menu on the HIM interface of the computer (48) and started the camera thanks to its own man/machine (HIM) interface (18), the image software (45) of the camera controls the initiation of the reading process of the wide-field (38) and small-field (39) sensors. At the same time, it triggers the LED lighting (15), whether specific or not, depending on the selected menu. This process will also cause the accelerometer (52) to start, which will send its information as a continuous or discontinuous stream to the picture software 1 (45) throughout the process, thus assisting in a correlation of the pictures, and which may at any time substitute one of the sensors, should it fail during the clinical action. The optical system (38) of the large field (20) will allow the image software system to know the field depth and to adjust, if we do not implement liquid lenses, the control (42) itself, adjusting, thanks to a micro-motor (22), the field depth of the optical system (41) of the small field (19) on the oral structures (21). Each of the two images will be captured by the CCD of the large field (38) and of the small field (39). They will be converted into digital data by the A/D converters (43 and/or 44) and/or arrive in analog form on the video control screen (49).

If the hardware supporting the image software 1 (45) uses too large a volume to be located in the camera (1), the second part of this image software (45) will be relocated in a standard (5) or dedicated (6) computer.

The information proceeding from this processing, as described later in this detailed description, will be addressed by all the nowadays known channels (51) capable of performing their processing, whether for diagnosis or for the CAD/CAM. This will be done using a modem (50) that will send its information, in both directions, by wired channels (internet and Ethernet, Wifi or telephone).

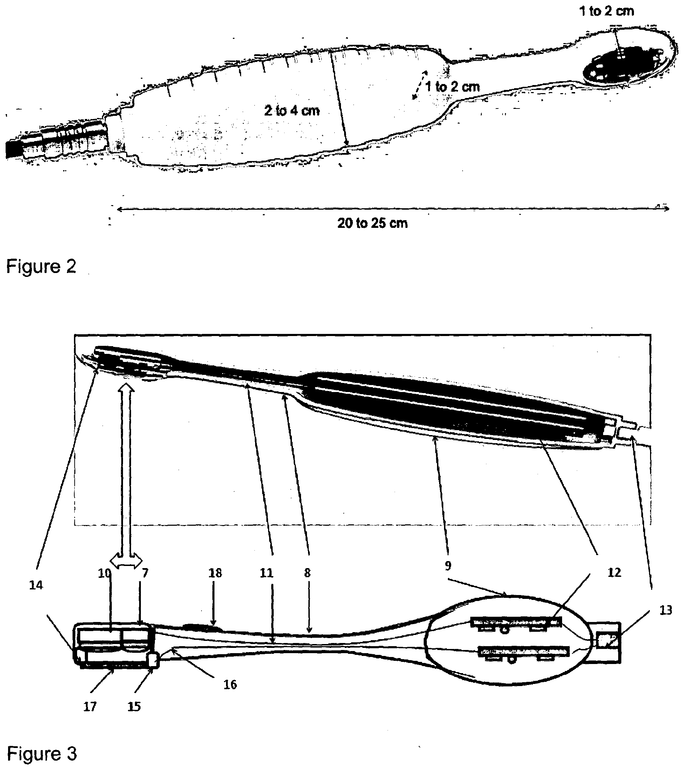

For the detail of each part of this invention, we will refer to Figure 2, which shows a dental clinic option in its functional aspect. In order to easily record an intra-oral picture, a 3D reading camera should be little voluminous. Unlike all the known systems, the present configuration enables us to have a very small-size 3D color camera, since its dimensions are between 20 and 25 cm, and has a body that is large enough to ensure a good grip (for example 2 to 4 cm) and a thickness that does not exceed for example 2 cm. It is an extended with an arm of 5 to 6 cm, which permits to pass the stage of the lips when recording an impression deep in the mouth. The reading head contains, in a non-hurting ovoid shape, for example 1 to 2 cm thick, aprox. a 2 cm width and a 3 cm length, the complete optical system, the LEDs and the CCD/CMOS sensors.

The cross-sectional view in Figure 3 permits us to better detail the components of this camera. In this configuration and this is not restrictive, we have a cross-sectional view showing the head of the camera (7), the arm (8) permitting its insertion into the mouth and the body (9), often outside of the mouth. The head has the cross-section of the optical assembly, here comprised of two optical systems (10) comprising three units (the lenses, eventually the system for adjusting the focal length (22) and the 2 CCD or CMOS sensors) connected to the image connection card (12) via a preferably shielded cable (11), in order to avoid interferences harmful to the quality of the information being transmitted. This card will itself be connected to the computer (5) or to the specific casing (6) through the specific connector (13) depending from the camera (1). This same longitudinal cross-sectional view permits to identify the LEDs placed towards the optical system (14) inside the head protected by the protective glass (17) and/or at the periphery of the optical system, outside the latter (15). A button (18) permits to activate the picture recording, when we do not use the foot pedal. Using a picture-recording system without any offset allows us to take this 3D image with the button without any risk of blur that could be created by an involuntary movement.

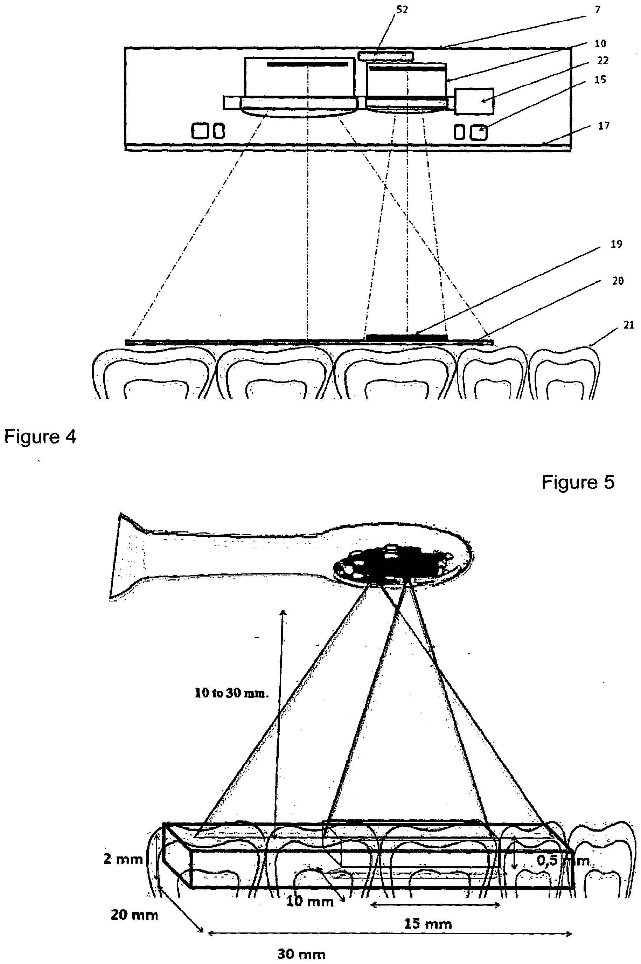

Figure 4 illustrates more accurately the basic principle of the present invention application. We see the schematic representation of the head of the camera (7) and the two different optical systems (10). These systems are comprised, from the bottom to the top, of the focusing and the image-transmission lenses and the CCDs/CMOS. These lenses are shown without focal adjustment system. If we use traditional lenses, it will be necessary to have a focal-length adjusting system (22) permitting to scanning in "z" a field with a 1 to 5 cm field depth.

Advantageously, the lens will be of the liquid type (Varioptic - Fr) or of glass or molded glass/plastic with a pupil on the input face.

The focal length will advantageously be between 0.5 and 5 mm, in order to meet the requirements of large and small field in the limited environment the oral environment represents.

The white and blue LEDs (15) are arranged around the optical system, immediately behind the protective glass (17), whether heating or not. They will preferably be specifically selected based on the desired type of lighting color.

It should be noted that there is no structured light projection, but two areas visualized by the optical system and the CCDs.

Advantageously, the narrow and accurate area (19) is completely included in the less accurate wide area (20) of the teeth measured by optical impression. As we can see, one of the advantages of this method is to include the accurate area in the general area, which largely facilitates the correlation of the two stereoscopic pictures. This also reduces the uncoded areas, since what one camera will not record will be read by the second one. The mere movement the camera will correct the eventual lack of coding.

Eventually and preferably, the narrow area can also be partially included in the area for purposes of industrial design and size. In this case, the narrow accurate measurement area will overlap the less accurate widest area.

Eventually and advantageously, in order to facilitate the reading of the accurate and narrow area, it is possible to add a displacement motor so that the narrow area quickly scans the entire wide area during the recording of pictures. The displacement motor may use all the techniques of displacement of the lenses.

Eventually and advantageously, this narrow area may be of variable zoom, which allows the operator to vary the desired accuracy in this narrow area between 1 and 20 pm, while benefiting from the large reading field in the wide area.

This stereoscopic camera is comprised of one or several unitary or multiple sensors, two in Figure 4, in a predetermined position, which ca be CCDs or CMOS, for example of 2 megapixels at 2.2 $\mu$m, (25 to 500 images / second) defining, by their renewal, the reading speed, thus the speed of recording of successive impressions permitting a static or dynamic reading, as we know for a photo camera or a video-camera. We can thus have a dynamic view by moving over the area of analysis, unlike with the profilometric phase systems that require a minimum of four pictures for extracting the relief, the system used in the present invention only requires a single frame or a double frame at two levels of accuracy, avoiding any movement in the measurement, or the integration of the information on the sensor is immediate and simultaneous.

It is also comprised of an optical assembly having one focal length or at least two different focal lengths, which can ranging from a numerical aperture (NA) of 0.001 to 0.1, and permits to transmit to the sensor(s) of the camera, without distortion, the data visualized on the two or several operatory fields. For example, for the intra-oral pictures, in the example shown in Figure 4, these fields can be described as follows;

a. one of the fields covers a large surface, but with a lower resolution, for example and this is not restrictive, of 20 $\mu$m (NA: 0.0125, i.e. a focal equivalent of F/8) over a field of 30x20 mm.

b. the other field is smaller, but more accurate, for example and this is not restrictive, with a resolution of 10 $\mu$m (NA: 0025, i.e. a focal equivalent of F/4) over a field of 15 $\times$ 10 mm. The field depth is small, a series of picture recordings with a variable depth is foreseen.

c. The small field is fully included in the large field, at all levels, whether centered or not, in order to detect the data for the generation of the three dimensions of the object (x, y & z) and to facilitate the real-time correlation between the accurate views and the general larger-field views.

d. The objective can be comprised of several glass or molded glass/plastic elements, the adjustment being performed by a micro-motor.

Eventually and advantageously, this adjustment the field depth on the teeth will be carried out using a liquid lens, in order to ensure a perfect adaptation based on the proximity of the intra-oral surfaces and to avoid the use of a micro-motor.

Eventually and advantageously, it can also be comprised of a lens, for example a thermoplastic lens referred to as "free-form" comprised of a flat top surrounded by n asymmetric facets ensuring, in one picture recording, the visualization of the oral environment according to n different viewing angles. The faceted portion is oriented towards the sensor and the flat side towards the oral environment. The sensor will receive n slightly different images with views from a different angle depending on the angle of cut of the facet with respect to the flat surface. Thus, in one single recording of pictures is possible the capturing and digitizing of n instantaneously correlated stereoscopic views of different surfaces, avoiding the addition of a second sensor and a second optical system.

Eventually and advantageously, if we have a single sensor, no longer the predetermined position of the sensor all the views, as we have seen previously, but the sequences of successive captures will define. The displacement movements correlated with a sequence of automatic picture recordings will define the different planes of picture recording. For example, the first image will be recorded at time T0, then a slight shift, which will lead to a change in angle of viewing, will be followed by a new recording at time T0 + 1 second (for example) and so on.

Eventually and advantageously, an accelerometer, a gyro or a 3D magnetometer (52) will be installed near the CCD/CMOS sensor, in order to assist with the correlations and to compensate for an eventual failure of one of the sensors. According to an embodiment of the present invention, in order to avoid any interruption in the clinical action or to replace one of the fields (large or small as the case may be), it will be for example a 3D accelerometer with a frequency of acquisition higher than or equal to 50Hz, an interval of +/- 10g and an accuracy lower than or equal to 3 mg. Eventually and advantageously, the general information on the field depth will be indicated by one of the sensors, for example the wide-field sensor, so that the focal length of the other, small-field sensor is prepositioned in an area close to the reality analyzed by the first, for example-wide field sensor.

Figure 5 shows the volume measured in the mouth of a patient. The small volume, in which the dentist can move his camera, considerably limits the possibilities of having both a wide field and a high accuracy. With the new concept introduced here, and sticking to the laws of optical physics, it is possible to measure a volume of 20 x 30 mm and a field depth of 2 mm with an accuracy of 20 $\mu$m at the level of the wide field. The narrow field limits the volume to 10 x 3.5 x 0.5 mm for an accuracy of 10 $\mu$m. This is given only by way of an example and can vary significantly depending on the qualities of the optical systems being used. These values are consistent with the requirements of an optical impression in the mouth for making good prostheses and good diagnoses.

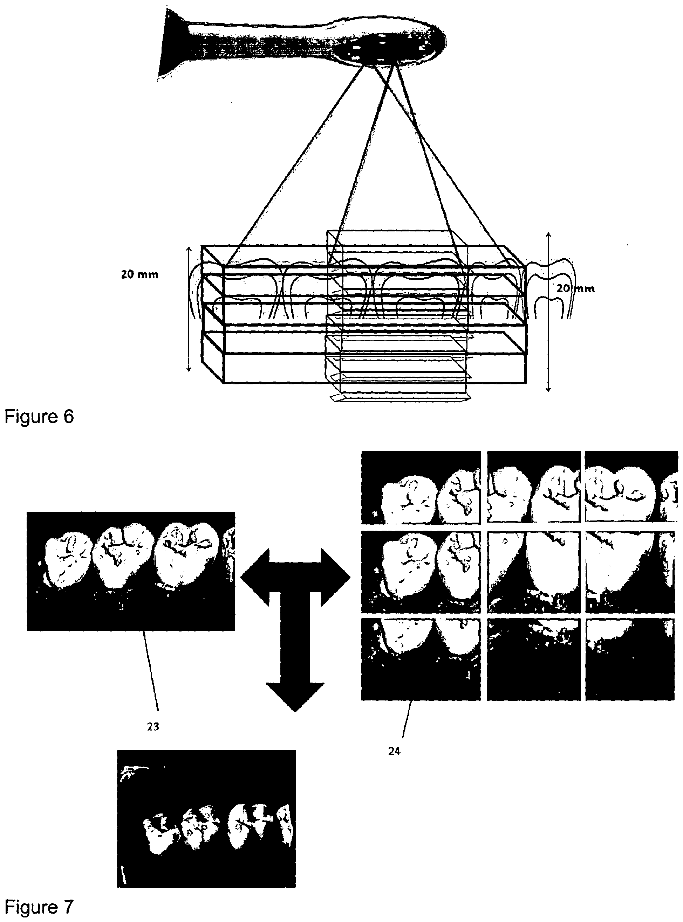

The field depth is insufficient, but it is laid on by the proximity of the teeth with respect to the optical system laid on by the space between the upper teeth and the lower teeth. In order to solve the problem of field depth, a series of picture recordings is provided for in Figure 6, by varying between 10 and 20 times in the accurate area and between 5 and 10 times in the wider area. This ensures accuracies within 10 $\mu$m (small and accurate narrow field) and within 20 $\mu$m (less accurate wide field) with a field depth between 10 and 30 mm, which is sufficient in dentistry.

Eventually and advantageously, these movements in field depth in the narrow field and in the wide field can be synchronized or not depending on the needs of the recording of optical impression. As wewill see in the software processing, this adjustment can be limited, since the CCD/CMOS can recognize whether the collection of information is unclear or not. This provides an information on the position of the teeth with pre respect to the optical system and enables an automatic-adjustment of the field depth. This also provides the advantage of limiting the scanning in depth and of limiting the successive picture recordings.

In Figure 7 we have the representation of the area scanned by the wide field (23) and by the succession of pictures of the accurate and narrow field (24). As we can see in the example given, ten pictures are sufficient to cover an entire field with an accuracy of 10 $\mu$m.

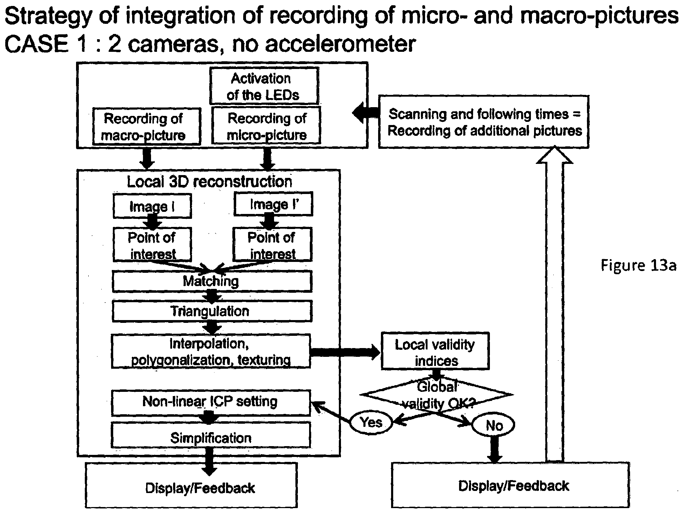

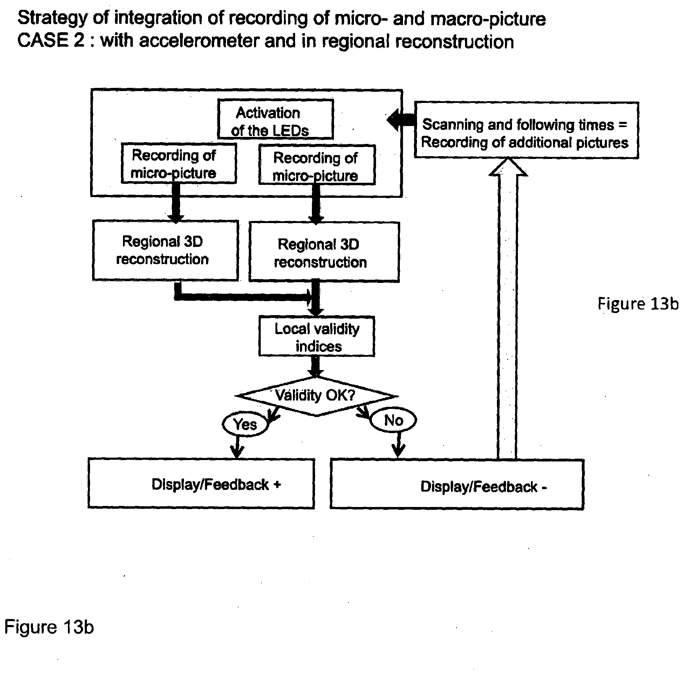

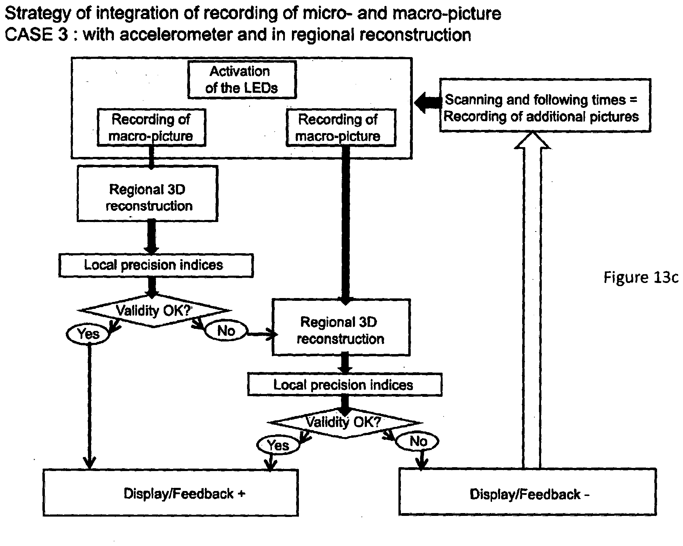

In fact, the dentist will position its accurate view on the central area requiring oral maximum accuracy. This area can be the finishing line of a preparation, but also, as we can see in Figure 7, the grooves and the cusps of the teeth. As will be presented later in the description of the "software", in particular in Figure 13 (stacked surfaces strategy), a judicious use of this high-precision area largely contributes to a high-fidelity reconstruction. The area common to both cameras is used for reconstruction and largely benefits of the level of details provided by the accurate field. On the other hand, by moving the head randomly, and thanks to the high frequency of acquisition of images, the user has a great chance to cover the whole area to be reconstructed by the part common to both cameras. Finally, should an area exhibit insufficient accuracy, visual feedback will be provided to the user, who can then focus the accurate field on this area, in order to achieve sufficient accuracy.

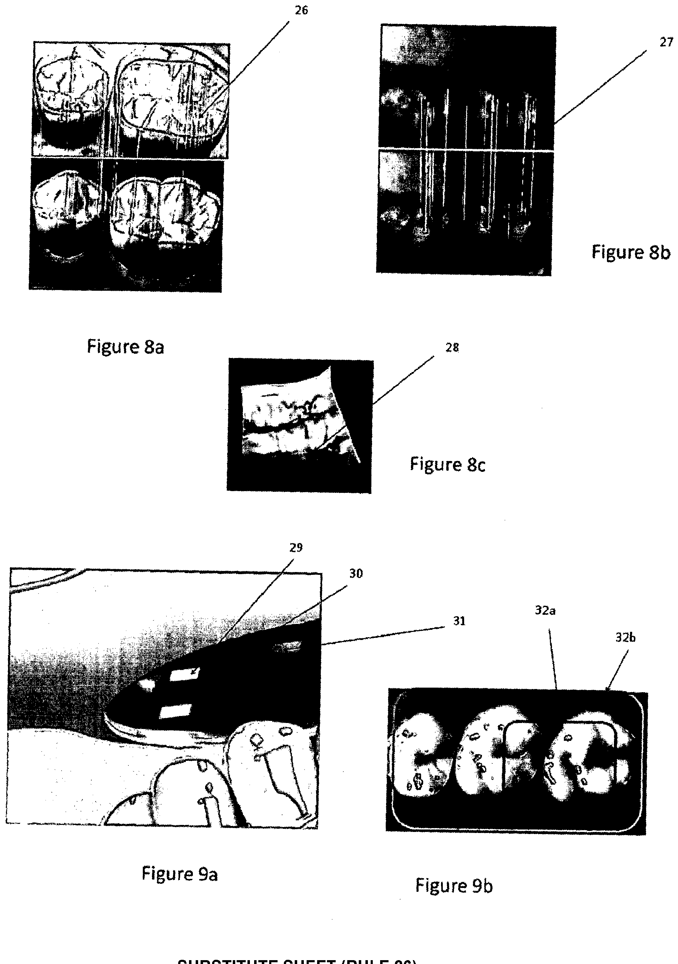

As can be seen in Figures 8a, 8b and 8c, a 3D stereoscopic view is possible when it is possible to correlate homologous dots found in each of the pictures recorded together or with a slight time shift. Figure 8a shows the automatic determination of the homologous dots in two occlusal and lingual pictures of the same teeth on a dental plaster (Figure 8a - 6). This automatic determination is possible with the software, which is an integral part of an embodiment of our invention. The lines that we can see unit identical and homologous dots identified in each of the two pictures. The same representation can be made on an intra-oral view (Figure 8b - 27) thanks to the software system.

Eventually and advantageously, the "software" permits this automatic identification of the area of focus in the area of field depth, while noting that everything happens for areas outside the field as if they had been subjected to a low-pass filter with respect to areas inside the fields therefore, the local power spectrum has a softer slope. The power spectrum is thus calculated in "patches" p of the image (typically a 20*20 pixel square area), the decreasing slope $\alpha p$ of which is approximated according to a decreasing exponential model. Then, the ratio $(\alpha p - \alpha 0)/\alpha 0$ is calculated, where $\alpha 0$ is the decreasing slope for the entire image. Is this ratio below a certain threshold adapted to the image, then the patch is considered outside the area of focus.

The result is a representation of a scatter diagram arranged in space (Figure 8c - 28), a part of which is very accurate (less than 10 $\mu$m}.

Eventually and advantageously, this representation as a scatter diagram is also performed thanks to the 3D reconstruction techniques described in Figure x.

Eventually and advantageously, this representation can also be made by a dense, polygonalisee and textured representation close to the actual visual representation, at the Bezier surface, by Radial Basis Functions, by NURBs, or by wavelets.

In this case, the software will proceed as described in Figure x, in order to perform this modeling. Schematically, the sparse scatter diagram generated by the 3D reconstruction (Figure x) is interpolated using the technique described in figure y. This technique has the advantage of densifying the scatter diagram and of modeling it by means of soft Radial Basis Functions type curves. (without loss of generality, the modeling can be performed for example, and this is not restrictive, by Bezier curves, by Radial Basis Functions, by NURBs, or by wavelets.) Once the surface model is applied, polygonalization occurs by means of a conventional technique (for example, and this is not restrictive, Bloomenthal technique, ball pivoting, Poisson reconstruction), then a texture as described in Figure z is calculated and applied.

The advantage of these modeling methods in real time or almost in real time is that they permit, starting from a stereoscopic view, an immediate 3D representation on the practitioner's display screen. He can vary the orientation and zoom digitally on all or part of the impression, in order to verify and/or validate his work for the following part of his clinical operations.

Fig. 9 shows the LEDs providing sufficient light for a good stereoscopic recording. In order to achieve an accurate and complete measurement, it is necessary to have a good lighting of the scene. The question is not at all to project structured light, but only to light the scene in a relatively dark mouth.

Eventually and advantageously, the lighting will be LED lighting for powers that can vary between 10,000 and 500,000 lux of white light and between 5,000 and 300,000 lux of blue light.

That is why a few LEDs are sufficient. In Figure 9a are shown two white LEDs (29) among the eight that are necessary to achieve 200,000 lux of white light and 1 blue LED (30) among the 4 blue LEDs that are necessary to achieve the 100,000 lux of blue light.

Eventually and advantageously, other LEDs will be added which have an unstructured light, but with the exact characteristics in terms of purity (consistent or not), of type (color) and intensity (power). In Figure 9a is shown, for example, and this is not restrictive, a green LED (31) permitting to develop some functions of assistance to the diagnosis on a 3D image, transferred onto our 3D surfaces.

This is the more interesting as since we are not using structured light, it is always possible to perform real-time color analyses in the mouth of the patients, both at the level of the mucosa and at the level of the mineral structures of the tooth or the prosthetic reconstruction materials.

Eventually and advantageously, the light will be chosen so that it can highlight mineral or organic carious fractures or damage in the crystal of the tooth. This is particularly interesting because the display will not occur on 2D images, as presently known, but on structures shown in 3D highlighting the areas to be analyzed, diagnosed or treated. This also allows the practitioner to follow up the quality of his work and to be sure, on 3D images, he has properly treated the highlighted disease.

Eventually and advantageously, this permits to highlight fractures in the restorative materials (as for example a slit in the zirconia ceramics) and to assess whether a new intervention on the reconstitution is necessary.

Eventually and advantageously, in addition to diffuse LED light, in order to assist the practitioner in knowing where the high-precision reading is located (narrow field in the wide field), the projection of a target (Figure 9b - 32a) surrounding this specific area is eventually foreseen.

Eventually and advantageously, other LEDs will be added, which have a non-structured light, but with the specific characteristics in terms of purity (consistent or not), type (color) and intensity (power). In Figure 9a is shown, for example and non-restrictively, a green LED (31) permiting to develop some functions of assisting to the diagnosis on a 3D image, transferred onto our 3D surfaces.

Eventually and advantageously, the projection of a frame surrounding the wide field (32b) is provided for, which avoids the practitioner from following his scanning on the screen during the recording of an impression in the mouth.

Using these blue and/or white LEDs has the advantage of permitting an easier search for homologous points and to determine a higher number of them on a tooth that has a crystalline and slightly penetrating structure. Eventually and advantageously, though the penetration of a diffuse LED light is not comparable to that of structured light projected on a surface of the tooth, the blue light will be used to make them look more chalky, avoiding the use of a covering layer referred to as coating.

Eventually and advantageously, the lighting system with LEDs of various wavelengths or colors, the mix of which will be chosen, for example, so as to create fluorescence or phosphorescence effects in the crystals of the tooth or in some parts or pathologies of the gum. This will further promote the display of the surface of the mineralized tissues in the blue or the UV, since a fluorescent tooth tissue has a particularly "mat" aspect, which avoids the surface or paint deposition referred to as coating.

This same application finally allows us to penetrate into finer gum areas, such as they exist in the dental sulcus. This permits the operator to have a view on the emergence of the tooth through the gum. Likewise, the choice of a judiciously selected complementary color, for example, among the red, permits to reduce the harmful effects of blood and saliva and facilitates the recording of an optical impression.

Advantageously, these LEDs will have a variable power and color, in order to light, at low power, the measured surface or, at high power, to cross some small thicknesses of the epithelial tissue.

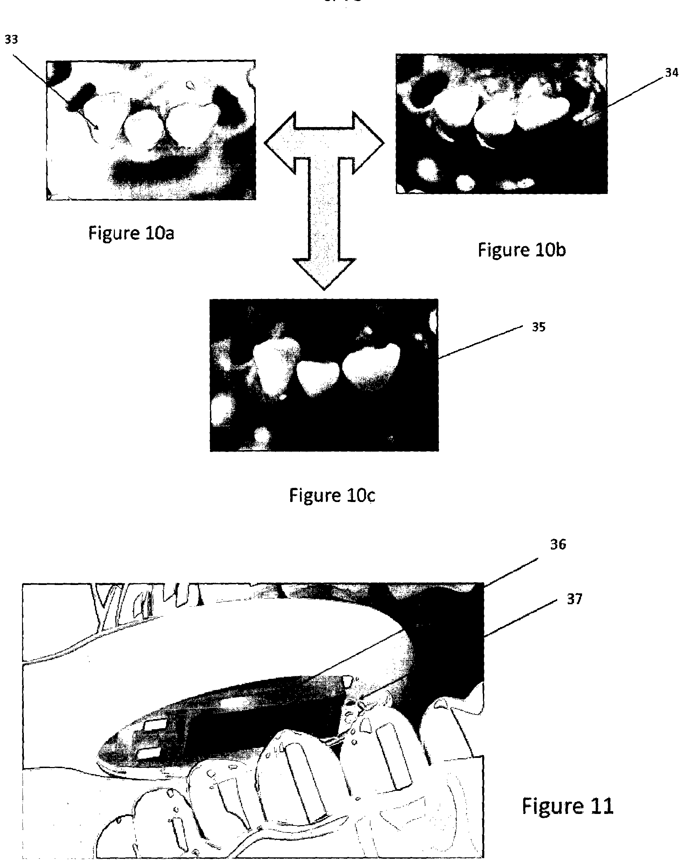

Through the mounting as provided for in this method, as Figures 10a, 10b and 10c show, a reading in white light is provided for, in order to have the exact color of the mouth environment (33) and eventually the addition of a picture recording in complementary light, for example and non-restrictively in blue light (34) or an association of the complementary light and the white light (complementary blue at 35).

Eventually and advantageously, one or more of the color components added to the white light will be subtracted, in order to arrange and represent on the screen and in real time the real color of the measured oral environment.

Eventually and advantageously, this choice of the LED color can be predetermined or automatic. If the scatter diagram is insufficient during a reading in white light, the system automatically (or manually) activates the complementary LEDs, for example the blue LEDs, and the system records again the same picture. The addition of the blue and white pictures multiplies the chances of increasing the information on the surfaces and the search for homologous dots.

Eventually and advantageously, these LEDs can also have a predetermined wavelength permitting to highlight the natural anatomic elements (bottoms of furrows or color areas differentiating tumors, gums or tooth shades) or markings made before the recording of impressions and made by means of specific and predefined colored markers.

These markings can advantageously be objects of different shapes placed in the measured area, glued or accommodated for example on the teeth, in the spaces between the teeth or on the implant heads, in order to facilitate the correlation of the pictures, but also in order to know the exact spatial position of these predefined marks.

In the case of implants or dental canals, this will permit to know some inaccessible areas during the optical reading. The identification of the mark and a priori knowledge of the carrying shape will permit to derive the shape and the spatial position of the hidden part.

The light combinations permit to highlight details on the areas with a weak texture, which do not appear under "natural" light. An optimal combination will be provided to the user by default: however, several pre-established combinations (which can highlight the markings, for example) will be provided.

The light combination permits, on the other hand, to have additional information for each spectral band. Thus, when we will present the algorithm for searching optical traces in figure x, the processing is not performed on the global image, but in parallel on the three spectral bands. The optical traces used for the 3D reconstruction result from the combination of the traces obtained for the three spectral bands.

In Figure 11, two additional functions required in the mouth are shown. Very often, during a recording of an optical impression, three optical elements that can degrade the information are avoided. They are blood, due to the preparation of the tooth, saliva that naturally flows in an open mouth, and mist that appears on a surface colder than the mouth.

For this reason and for reasons of comfort and accuracy, it is foreseen to associate with the camera, in the reading head, a spray of air or liquid, of which can be seen the aperture (37), which is directed towards the reading area. This permits to evacuate saliva or blood during the reading.

Likewise, the glass protecting the optical system and the LEDs in the head of the camera, is designed as a heating glass, for example between 20 and 35°, depending on the seasons, so as to limit the deposition of mist on the protective glass.

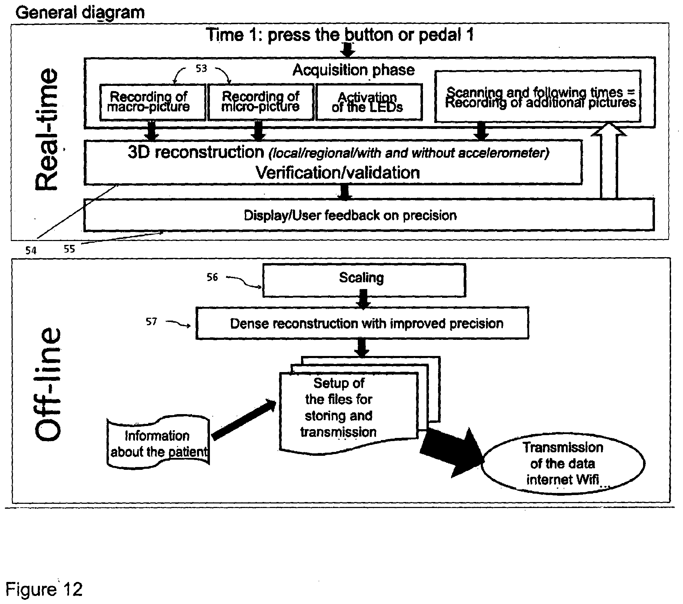

Figure 12 shows the general diagram of the software portion. This diagram permits both to provide a real-time 3D reconstruction during the acquisition and to ensure spatial high-fidelity of the final model.

A first reconstruction is performed in real time and sequentially: when images are acquired (53), a regional 3D reconstruction (54) is calculated (from this only pair - if two cameras - or with a few preceding pairs - if a single camera) then added to the global reconstruction as it was before the acquisition of this pair. The reconstruction is instantly displayed on the screen (55), eventually with annotations on its local quality, enabling the user to visually identify the areas in which a second pass would eventually be necessary. The sequential reconstruction is continued until the user completes the acquisition of images.

Once the acquisition is complete, we proceed to the final adjustments of the reconstructed 3D model: enhancement of the accuracy of the model and estimation of the scale factor. The total duration of the final adjustment does not exceed 5 minutes.

First of all, the 3D reconstruction may require a scaling (56) when the images were acquired from a single camera. The estimation of the scale factor to be applied to the reconstructed 3D model is performed by means of a filter, for example, and this is not restrictive, a Kalman filter, and uses both the measurements for example, and this is not restrictive, from the accelerometer and those from the images (relative positions of the cameras with respect to each other).

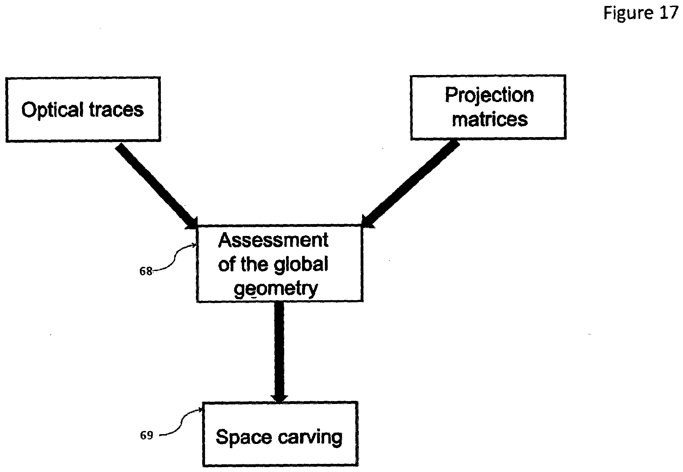

Furthermore, the real-time 3D reconstruction is refined in order to increase accuracy (57). The precision-gain technique is detailed in Figure 17.

Figures 13a, 13b and 13c schematically show how the pictures acquired from the two cameras can be used. To this end, three ways of operating, and this is not restrictive:

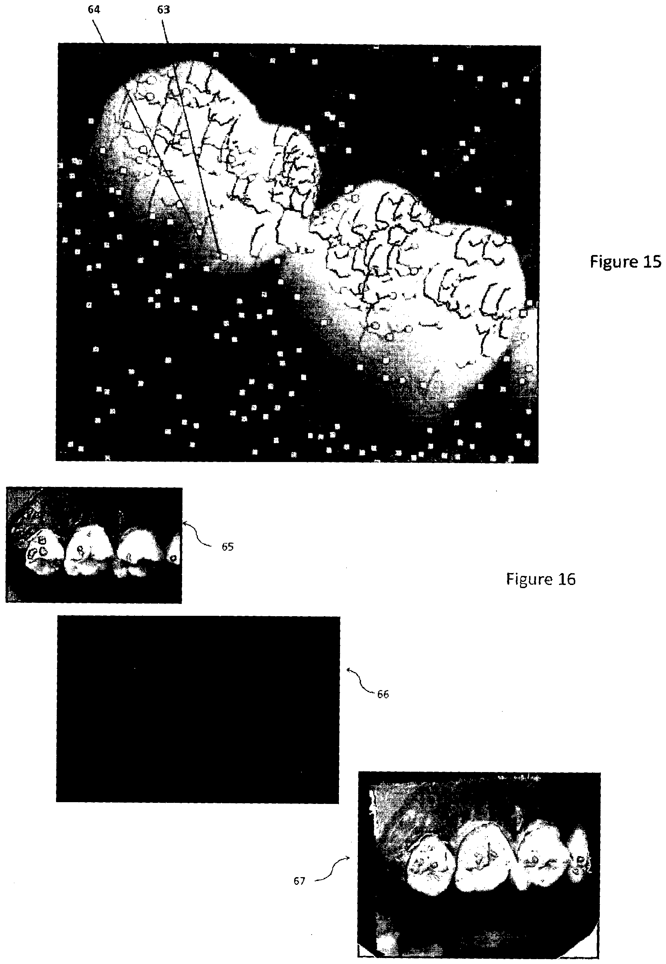

- Figure 13: When a pair of images is newly acquired by the two cameras, we look for the optical traces (dots of interest and correspondences) among the two images (algorithm shown in Figure 15). The corresponding dots then permit, by triangulation, to calculate the corresponding 3D dots. Triangulation is extremely simple in the case of two cameras, since we are in a calibrated configuration, in which we know the intrinsic (focal length and distortion) and extrinsic (relative positions of the cameras with rest to each other, by construction of the camera) parameters.

The 3D scatter diagram generated is then interpolated, polygonalized and textured (algorithm shown in Figure 16). A validity index q (57) is then calculated for each element (for example, and this is not restrictive, triangle or

$$q = \frac{216 \circledast \sqrt{3} \circledast V^2}{(a+b+c+d)^2}$$

tetrahedron) of the polygonalized 3D reconstruction. We will chose $\quad$ (v = volume, a, b, c, d = length of the sides of the tetrahedron, for example, and this is not restrictive). If, at a point, this index is lower than a certain threshold, the reconstruction element is labeled as invalid, which will permit a real time visual feedback to the user during the phase of display, so that the user can acquire new pictures in this area and thus obtain a sufficient quality. A global index of validity of the reconstruction generated by the pair of images is also derived, by calculating the percentage of invalid elements compared to the total number of reconstruction elements. If this percentage is lower than a certain threshold, the generated surface will not be integrated into the reconstruction.

The generated surface, if valid, is integrated into the partial reconstruction for example by resetting, and this is not restrictive, of the non-linear Iterative Closest Point type followed by a simplification (removal of redundant 3D dots or outliers). Eventually and advantageously, the integration into the partial reconstruction can be done by performing a tracking of the relative positions of the cameras by an algorithm similar to that shown in the following figure.

Finally, the reconstruction phase is followed by a phase of display.

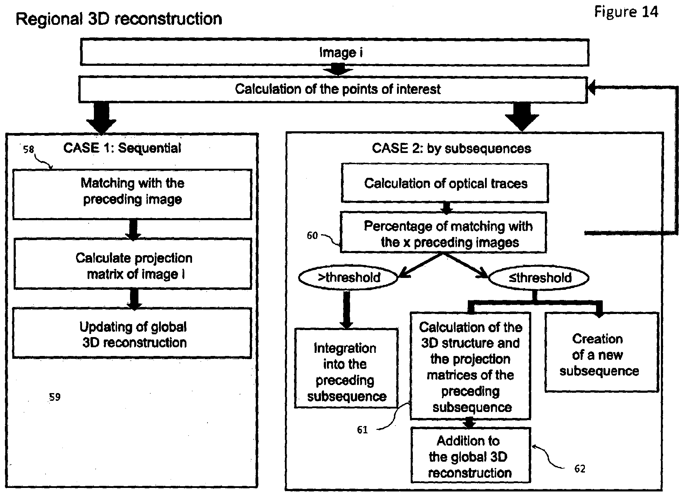

» Figure 13b: Alternatively, the images from the two cameras can be used independently. Two regional 3D reconstructions can be calculated independently for the wide-field camera and the small-field camera, thanks to the algorithms shown in Figure 14. Since the small-field reconstruction is calculated based on images that integrate into a fixed position in the large-field images, it can be directly integrated into the large-field reconstruction. The end of the algorithm is then similar to the case shown in Figure 13a.

• Figure 13c: Alternatively, the images of the small-field camera can be used only sporadically. During the acquisition, then they are stored, but not automatically processed. The reconstruction is carried out only from the wide-field camera, thanks to one of the algorithms of Figure 14, then the local quality indices are calculated. For the invalid elements, one looks through reverse projection to which portion of the large-field 2D image they belong, then one looks in the small-field image database whether some images (typically some ten images) cover this area. A local reconstruction is then calculated based on these small-field images, then the validity indices are re-calculated. If the latter are above the threshold, then the small-field reconstruction is integrated into the large-field one in a way similar to Figure 13b.

Figure 14 details the two strategies usable for reconstructing the 3D model from a single camera. The complexity of the algorithms used in this case results directly from the freedom given to the user to use the system without any constraint. Thus, the movements of the system cannot be predicted; in other words, when the picture recordings are acquired, we cannot know a priori from where these pictures have been recorded. It is then up to the algorithms to find the specific spatial organization of the pictures, in order to ensure a faithful reconstruction of the object.

- Sequential Case: We work in a protective geometry, which requires from the start of the acquisition to choose a pair of images serving as a geometrical reference. The choice of these first two pictures is essential to avoid falling thereafter into a problem of local minima. Among the first images of the acquisition, the initializing pair is selected such that:

◦ The number of matches between the first two pictures is at least 400.

a The distance between these two pictures is large enough: arbitrarily, we will wait for the data from the accelerometer that at least 5mm have been covered; otherwise (if the operator remains immobile), we will wait until at most 40 images have been acquired.

From these first two pictures, a first estimate of the geometry is performed;

a The optical trace is calculated between these 2 images (algorithm of Figure 15)

o The projection matrices P1 and P2 (representative of the spatial position of the cameras) are calculated from the matches by a conventional 5-point algorithm.

a The corresponding dots are triangulated, in order to obtain an initial estimation of the 3D dots.

◦ The geometry Is updates by self-calibration, in order to pass from a projective geometry to a nearly-metric geometry (within one scale factor).

◦ The generated 3D scatter diagram is then interpolated, polygonalized and textured (algorithm in Figure 16). The generated surface is the first estimate of the partial 3D reconstruction.

Then, the reconstruction is enriched thanks to any newly acquired picture i:

• the optical trace is complemented by calculating the dots of interest in this picture and by matching it with the previous picture (58).

• Knowing the correspondence with certain dots of interest in image i-1, and knowing the coordinates of 3D points that are projected onto these dots of interest, it is possible to estimate the projection matrix $P_i$, for example and this is not restrictive, by re-sectioning (59).

» Since all the projection matrices are now known until image i, we re-estimate the 3D dots linearly based on these matrices and the optical traces. In practice, in order to maintain the real-time constraint, we only work on the current picture and the n previous pictures (typically, n=3 or 4). The total geometry on these n pictures (projection matrices and 3D dots) is then refined by a non-linear algorithm for example, and this restrictive, of the Sparse Bundle Adjustment type.

• The total 3D scatter diagram is again interpolated by multiscale RBF, then polygonalized and texturized.

• The local indices of validity are calculated, and then follows the visualization phase.

• Case by sub-sequences: The sub-sequence strategy calculates partial reconstructions for sub-sequences of images, formed by isolating spatially coherent groups of images and having a large number of corresponding dots. One proceeds as follows:

o Sequencing algorithm: The video stream is divided into sub-sequences, referred to as regions, as the acquisition progresses, after calculating the optical traces. If the optical search occurs by tracking, a region ends (60) when the percentage of dots still in tracking phase drops below 70%; for the other optical search techniques, the region ends when the number of matches with the first image of the region is lower than 70% of the dots of interest of the current image. When the current region is closed, a new region is created and initialized with the new image being acquired.

◦ As soon as an area is closed (61), the relative positions of the cameras and the 3D dots corresponding to the optical traces found in this region by an factorization, for example and this is not restrictive, of the Tomasi Kanade type are calculated in parallel. The generated 3D scatter diagram is interpolated, then polygonalized and textured (algorithm of Figure 16).

◦ The geometries differ by region when this algorithm is used as is; the generated surfaces are thus not coherent in space. In order to bring all the regions in the same geometry (62), one should be careful to put some images (typically 3) artificially in common between 2 adjacent regions, which will permit to derive a transformation homography between pairs of adjacent regions. The homography is applied to each end of the generated surface, in order to integrate it into the global model.

• The local indices of validity are calculated, then follows the visualization phase.

Figure 15 shows an example of calculation of an optical trace by tracking dots of interest. The dots of interest of the current image are represented in it by squares (63), while the lines represent the positions of these dots of interest in the previous images.

The search for noticeable optical traces of 3D dots occurs by searching dots of interest in all the acquired 2D images, then by searching matches between the dots of interest of different images. Several schemes are possible:

• Optical Tracking of Angles: The general idea is to calculate noticeable dots (angles) in an image, then to track these dots in the following images without having to re-detect them. The tracking phase continues as long as a certain percentage of noticeable dots of the first image is still detectable (typically 70%) ; below this threshold, a new detection phase of noticeable dots is conducted on the following image.

The detection of angles occurs by calculating for any pixel (x, y) the 2*2 matrix

$$c = \begin{bmatrix} \sum_w \left(\frac{\partial I}{\partial x}\right)^2 & \sum_w \left(\frac{\partial I}{\partial x}\right) \circledast \left(\frac{\partial I}{\partial y}\right) \\ \sum_w \left(\frac{\partial I}{\partial x}\right) \circledast \left(\frac{\partial I}{\partial y}\right) & \sum_w \left(\frac{\partial I}{\partial y}\right)^2 \end{bmatrix}$$

, where I denotes the intensity in (x, y) of the image and W a surrounding of (x, y). Let's assume that $\lambda 1$ and $\lambda 2$ are the 2 eigenvalues of this matrix; if these 2 values are above a certain threshold (typically 0.15), the dot is considered as a noticeable dot.

For the tracking, we look, among 2 images i and i +1 and for each noticeable dot, the displacement $d=(d_x, d_y)$

that minimizes $\sum_w \left(I_i(x, y) - I_{i+1}(x + d_x, y + d_y)\right)^2$ . This displacement is calculated by $d=C^{-1}.b$,

C being the 2*2 matrix evoked above, and $b = \sum_w \begin{bmatrix} (I_i(x, y) - I_{i+1}(x, y)) \circledast I_i(x, y) \\ (I_i(x, y) - I_{i+1}(x, y)) \circledast I_{i+1}(x, y) \end{bmatrix}$ . Since this optical tracking technique is reliable for small displacements, the contingencies of large displacements are coped with by sequentially calculating the displacement d on a pyramid of images (from a largely subsampled version of the images to the original resolution).

The above-mentioned techniques are based on the implicit assumption that the stream of images is consistent, i.e. the displacement between 2 successive images is small, and 2 successive images are of sufficient quality to find a satisfactory amount of matching dots (at least 30).

As regards the displacement between 2 images, the acquisition of the images occurs at a conventional videostream frequency. We can therefore expect a very small displacement between 2 images. For a larger displacement that would result into an impossibility of finding dots corresponding with the previous images, a new region can be generated.

As regards the insufficient quality of an image (in the eventual case of a blurred image, for example), the matching phase acts as a filter, since it is clear that very few matching dots will be found. The image will then be stored without being processed, and one will wait for the next image that will have a sufficient number of matching dots.

• Unchanged dots + matching at least squares: The dots of interest are sought in the 2D images by well-known techniques, which look for dots that remain unchanged under change of scale and illumination. These techniques have the advantage of being capable of calculating morphological descriptors for each dot of interest.

The matching between dots of interest for a given pair of images is performed by searching for any dot of interest $x_{11}$ in image 1, the dot of interest $x_{12}$ in image 2 minimizing the distance at $x_{i1}$ at the least squares in terms of descriptors. In order to avoid false matches or outliers, the fundamental matrix F will first be calculated between images 1 and 2 (which binds the pairs of dots of interest by the ratio $x_{i1}.F.x_{i2}{}^t = 0$.

If, for a pair of potentially matching dots of interest $x_{i1}$ and $x_{i2}$ at the least squares, the product $x_{i1}.F.x_{i2}{}^t$ is larger than $10^{-5}$, the pair is rejected.

The search for an optical trace then occurs by transition during the acquisition of a new image. When acquiring image $I_j$, it is assumed that the calculation of the optical trace was performed for all previous images $I_1 \dots I_{j-1}$. The dots of interest $I_j$ are then calculated, which are brought into correspondence with image $I_{j-1}$. The optical traces are then complemented by transition, whereby it should be noted that if $x_{ij}$ is in correspondence with $x_{ij-1}$ and $x_{ij-1}$ is in correspondence with $x_{ij-3}$, then $x_{ij-1}$ is in correspondence with $x_{ij-2}$.

- Strong gradients + matching by correlation: As dots of interest of an image are considered all the dots where the variations in intensity are important. In practice, for each dot of the image considered is calculated the standard deviation of the intensities in a 20*20 pixel surrounding around this dot. If the deviation is above a certain threshold {typically in the range of 10, for intensities coded on 8 bits), then the dot is considered as a dot of interest.

The search for matches between 2 images at the level of their dots of interest occurs by a correlation technique, for example and this is not restrictive, of the Medici type (French Patents filed on 29.03,2005 EP1756771 (B0453) and EP0600128 ( BO471)).

Figure 16 shows three simplified steps of the real-time 3D reconstruction algorithm. The reproduction (65) is one of the 2D images of the acquisition to be reconstructed. The reproduction (66) represents the scatter diagram generated by one of the algorithms for calculating the 3D scatter diagram. The reproduction (67) shows the partial 3D reconstruction calculated based on the reproduction (66) thanks to the algorithm for interpolating the scatter diagram, polygonization and texturing detailed below.

The 3D modeling follows three steps. In the first step, the 3D scatter diagram obtained by processing the optical lines is densified by calculating an implicit interpolation function f. Thanks to this implicit function, the 3D surface interpolating the points is polygonalized for example by means of the method, and this is not restrictive, such as Bloomenthal. Finally, each polygon is textured in a very simple way: by projecting the 3D points delimiting the polygon onto the images that generated these points, a polygonal area is delimited on these images. We then determine the average value of the texture of these polygonal areas, and it is assigned to the polygon.

The main difficulty resides in the algorithm used for interpolating and calculating the implicit function. This algorithm is optimally adapted to our use, because it permits a real-time interpolation and, unlike other interpolation techniques, it permits a dense interpolation from a very scattered initial diagram, which is very often the case when working with objects with little texture like the teeth. Below we explain the generic interpolation underlying this algorithm, then its use in practice in a multi-scale scheme;

- Generic Interpolation: Assuming that Pi represents the dots of the 3D diagram (after estimation of the normal $\vec{n}$ at these points), we will search for the implicit function $f: R^2 \to R$, based on RadialBasis Functions (RBF) such that the points X belonging to the surface are those for which f(X)=0. We choose f such that:

$$f(x) = \sum_{p_i \in P} \left[ g_i(x) + \lambda_i \right] \circledast \phi_\sigma \left( \| x - p_i \| \right),$$

with

$$\phi_\sigma(x) = \phi\left(\frac{x}{\sigma}\right), \phi(x) = \left(1 - r\right)^4 + \left(4r + 1\right)$$

The unknowns to be determined to explain f are thus the $g_i$ and the $\lambda_1$.

Estimation of the gi: Let's consider the point Pi and its normal $\vec{n_i}$, let's choose a system (u,v,w) such that u and v are perpendicular to the normal and w points in the direction of the normal. Assuming that h is a function of the form $h(u,v) = Au^2 + Buv + Cv^2$, we look in pi for the coefficients A, B and C so as to minimize the following quantity

$$\sum_{p_i \in P} \phi_\sigma \left( \left\| p_i - p_j \right\| \right) \circledast \left( w_j - h(u_j, v_j) \right)^2 ,$$

We then calculate gi (x) by $g_j(x) = w - h(u,v)$.

Estimation of the $\lambda_i$: Knowing that $f(P_i) = O \forall P_i$, we can estimate the $\lambda i$ by simply solving the linear system.

a Multiscale Interpolation: The generic interpolation is actually conducted on subsets of points, in order to largely improve the accuracy of the interpolation. We first of all construct a set $\{P_0,....,P_k\}$ as follows: the set $P_0$ is a parallelepiped including the set of points Pi. Between 2 successive levels k-1 and k, a subdivision of parallelepipeds into 8 small parallelepipeds made.

The function f is calculated by an iterative procedure. We start with $f^0$=-1, then we iterate on the sets $P_k$ by updating f:

$$f^k(x) = f^{k-1}(x) + o^k(x), o^k(x) = \sum_{p_i^k \in P_k} \left( g_i^k(x) + \lambda_{ki} \right) \circledast \phi_{\sigma^k} \left( \left\| x - p_i^k \right\| \right)$$

The $g_i^k$ are determined as described above on the set $P_k$, and the $\lambda i$ are calculated by solving the system

$$f^{k-1}(p_i^k) + o^k(p_i^k) = 0 .$$

The $\sigma^k$ are updated such that $\sigma^{k+1} = \dfrac{\sigma^k}{2}$, and the number of levels to be constructed is defined by

$$M = -\log_2 \left( \frac{\sigma^0}{2\sigma^1} \right)$$

. Figure 17 shows the 2 steps of enhancement of the accuracy:

- Global calculation of the geometry (68): In contrast to all the real-time 3D reconstruction techniques presented above, we use, at the end of the acquisition, a re-assessment of the spatial positions of the cameras and the 3D points based no longer on some images (fixed number of images if sequential strategy, region if sub-sequential strategy), but on all the images of the acquisition.

We therefore use an algorithm of the type Sparse Bundle Adjustment, with as the initial estimate the positions of the 3D points and the projection matrices of the cameras as they were at the end of the acquisition. The scatter diagram is finally densified by the interpolation algorithm evoked above.

- Space carving (69): Once the global 3D scatter diagram has been re-calculated, the global 3D reconstruction consists of a Delaunay triangulation of the diagram. This triangulation provides a much too dense set of polygons, not taking into consideration the visibility of the points. In order to segment this model and to extract only the visible information, we perform a graph-cut type segmentation aiming at minimizing the energy E= visibility + photo-consistency + surface, with:

    o Visibility: for each tetrahedron of the model is known from which cameras it was reconstructed. It is thus visible from this camera and no other tetrahedron should be located between it and the camera. Thus, for each tetrahedron, the term visibility counts the number of tetrahedra between it and the camera.

    o Photo-consistency: Let's assume that p (T) is a photo-consistency measure for a triangle T of the reconstruction. (Traditionally, we can take the average value of the differences between the texture of this triangle and the textures of the 2D points, which its vertices are derived from). The term photo-consistency energy to be minimized

$$E_{photo} = \sum_{T} p(T) \circ aire(T)$$

is equal to                                                     . In the care of the minimization per graph cut, we will minimize by adding to the graph, for each pair of tetrahedra sharing a triangle T, two nodes p and q with a weight edge $W_{pq}$ = p(T).

○ Surface area: we try to have a surface with an as small as possible surface area. We will minimize by adding to the graph, for any pair of tetrahedra sharing a triangle T, two nodes p and q with a weight edge $W_{pq}$ = aire(T).

The handling of such a system is extremely simple because its characteristics are deemed fixed and unchangeable by the operator, except the type of selected lighting, although this function can be controlled by a sequence of automatic actions leading to the desired diagnosis. To this end, the operator (dentist, dental technician or physician) has a computer showing him the operations the camera can carry out and permitting him to choose between one function and another one.

All or part of the treatment can occur at the level of the cards included in the camera, whereby the rest of the treatment can eventually be performed by a generic system (laptop or standard desktop computer) or a specific system including cards specifically dedicated to the application of processing, transmission and data display.

Thus, in "measuring" function, after having selected this mode of action, the operator starts the measurement, using a button located on the camera, or a pedal in communication with the computer, the camera or on the intermediate casing, after having positioned the camera over the area to be measured and stops it when the feels he has enough information. To this end, he stops the pressure, or presses a second time.

The camera is, in this case of picture recording in the mouth or on a plaster model, moved over the arch, in order to collect the color 2D information, x and y, on each of the sensor(s), which can be CCDs/CMOSs with or without accelerometers.

The software processing permits to calculate practically in real time the 3D coordinates (x, y and z) and the color of each of the points measured on x and y. We obtain a 3D file of a partial or complete arch in color.

The successive recordings of images, a real film of the area to be measured, permit a complete record of the information necessary for the digital processing of all or part of the object measured in the vestibular, lingual and proximal area. A slight light pattern permits to indicate the successive picture recordings to the operator.

The knowledge of all the points of all the surfaces of the two measured arches also allows the operator to re-record certain insufficiently accurate areas. These areas are identified automatically by the software by means of different real-time systems such as the existence of a lack of information on the scatter diagrams (wide detection) or the existence of aberrant dots with respect to their immediate vicinity (local detection).

This same detection can occur at the level of the modeling curves (Nurbs, radial basis functions, wavelets ....).

These areas will be marked with a color or by another method capable of drawing the clinician's attention. The latter will take again the camera and the identification of the new points with respect to the known points will permit to fill in the inaccurate spaces or areas. This operation can be facilitated by numbering the areas to be read again, a reading order to be followed, and/or the presence of a 3D accelerometer.

These data undergo, on the one hand, an analog-to-digital conversion and, on the other hand, are eventually processed in the form of a video signal directly usable in real time by the conventional display screens.

Having a colored image also allows the operator to have an automatic analysis of the dental (usually white) and gingival (usually red) areas, which is impossible with the current methods using the projections of structured light. Likewise, through positioning an index of known color he has the possibility of carrying out a discriminative analysis in order to identify objects in the image, but also their position (implant or screw heads, orthodontic brackets ...) or also to facilitate the correlation of the pictures (colored marks, lines on the object or selective colors such as the bottoms of furrows...)

This discrimination has another advantage at the level of the software. Since the current methods often do not have the color analysis, because of the projection of structured light, they have so-called "unrelated" surfaces, which disturb, even impede the automatic correlation of the pictures. They require a manual cleaning of the pictures, which operation is time-consuming and expensive. Being able to distinguish between the gum (red) and the teeth (white) will permit to remove the unrelated areas based on the color information. Thus, in an analysis surface of the preparations of the teeth, all red unrelated areas will automatically be deleted.

Finally, in the measuring function of our invention, the high accuracy of 10 $\mu$m is not always necessary and that of the wide field is sometimes enough (20 $\mu$m). In dentistry, the practitioner, who wants to carry out a diagnosis or an impression, in order to make a prosthesis or an implant, needs two types of approaches, a fast one, which provides him only with the necessary information (in terms of measured surface and provided accuracy), and the other one, a complete and accurate one. For example, making a crown on a mandibular molar tooth can be done by dental CFAO when the optical impression of the preparation area is accurate, complete and neat, when the optical impression of the opposing teeth provides at least the measures of the points of contact (cusps, furrows) and the arch forms, which does not require the same attention. Likewise, an impression for a device for straightening the teeth (orthodontics) will not require as much accuracy as the one for making a ceramic bridge on implant heads.

Eventually and advantageously, the present invention permits to select independently from each other wide-field or narrow field accuracies, thanks to the software implemented in image processing (Figure 1b). It is possible to quickly construct large-area color surfaces or, on the contrary, to construct narrow areas with high accuracy, by putting into operation only either one of the sensors, preferably associated with the accelerometer the function of which will be to replace the inactivated sensor. This substitution is not necessary, but is a supplement that guarantees the accuracy of the correlation of the pictures.

In the function referred to as "diagnosis", he selects on the computer the desired type of diagnosis, e.g. melanoma, and the camera will start a scanning with a wavelength corresponding to highlighting the areas of interest for the pre-selected wavelengths present on a 3D image. In addition, and through the 3D analysis of the object, the recovering of the measures over time will permit to better follow the evolution of said pathology. It is indeed recognized by the professionals that the study of a suspicious image can be made in 2D, but especially the evolution of its volume and its color serves as a reference for monitoring its dangerous character over time. Having a volume referred to a mathematical center (e.g. the microbar center) permits to superpose images on a center depending on the object, and not on the observer, in order to objectively assess the evolution of its volume, the color analysis being transferred onto a 3D form, which is not the case today with the methods performed on 2D surfaces or those using structured light or waves (OCT, scanner or MRI).

Likewise, thanks to the 3D color display of our invention and by selecting the "color analysis", the analysis of the color of the teeth will be transferred onto their measured volumes. This measurement will be done by colorimetry using 3 or 4 basic LED colors (RGB). Being able to have different LED colors, thus several wavelengths, we can approximate a continuous spectrum, without the risk of disturbing an structured active light. We will have a spectro-colorimetric analysis independent from the metamerism.

Advantageously and according to an embodiment of the invention, the LEDs can also play an important role in the correlation of the successive pictures (Figure 12) (85). Indeed, we know that there are methods based on the correlations of the pictures with marks placed in the measured environment or using the similarity found in the diagram itself, or even working on the fuzzy edge of the pictures. All these systems are complex, because they require either placing spherical marks in the area, which operation is complex at clinical level, or identifying areas often without any relief or with too an even condition of the surface.

Scanning with LEDs having a known wavelength with a color 3D imaging permits to simplify and automate this process. Indeed, a simple colored line or the sticking of a mark can be detected and displayed automatically if we have taken care to use a marking using a color that is complementary, identical, additive or subtractive of the wavelength of one (or several) of the scanning LEDs (79). The detection will thus occur through a simple chromatic highlighting of any mark whatsoever. This marking, which is always in the same position on the object, regardless of the angle or zoom of our optical impressions, will serve as a correlation reference.

Advantageously and according to the same principle, it will be possible to track the mandibular movements by placing our camera in the vestibular area of the jaws of the mouth. We draw red-color lines on the upper jaw bone and the lower jaw bone, and this is only a non-restrictive example, and then we film the movements of these two jaw bones, in a vestibular view, from the start to the end of the movement. The camera takes pictures in which a scatter diagram moves (the lower jaw bone) relative to the other scatter diagram (the upper jaw bone, which is in principle considered immobile). Since our marking belongs independently to each jaw bone, our system will only track the movement of the colored markings, highlighted when the red LED is lit (in our example and this is only an example). Since this same marking exists at the time the optical impression made separately of the upper jaw bone and the lower jaw bone, the correlation software will use this colored marking not only for correlating the images of each one of the jaw bones, but also for displaying the movements depending on the fourth dimension, the time.

This operation can be performed without using a marker, but only through the identification of the scatter diagram common to the upper and lower jaw bones.

It is also possible to measure the position in occlusion and the displacement of an arch with respect to the other one. To this end, the camera is positioned laterally, with clenched teeth, in order to take the coordinates of the points visible on both arches, usually located on the labial surfaces of the teeth.

Since the points detected in the vestibular pictures are common to the individual pictures of each of the arches, it is possible to correlate all the points of both arches taken individually and to so have all the points in occlusion, including the inaccessible areas in the vestibular view, with clenched teeth.

We then have three types of point files, the file of the upper arch, that of the lower arch and that of the two arches in occlusion referred to as static occlusion.

If we position the camera for a vestibular view, with clenched teeth, and we ask the patient to move his teeth, we will have a fourth file corresponding to the temporal displacement of the upper arch with respect to the lower arch. It is enough to follow over time the movement of the points identified in the vestibular view. This will provide the information on the dynamic movements in occlusion.

This same operation can be performed using a laboratory patch or articulator. The camera will follow the displacement of the vestibular points detected on the plaster models placed on the articulator.

Starting from this static analysis of the occlusion, it is possible to position our virtual models in a virtual articulator as introduced in Chambéry in 1985 and to follow the dynamic movements by adjusting the essential data, which are the condylar inclination, the Bennett angle and other essential information given by a face-bow.

We can advantageously use the points of the 3D analysis resulting from our invention in order to properly position the virtual model on the virtual articulator and/or we can use the marking points as defined in our patent EP 0373077 or our patent application EP 93.913173.6.

Based on this static and dynamic occlusion measurement, we can use the method described in our patent EP 0369908 (US 5,143,086) "device for measuring and analyzing the movements of the human body or part thereof". This will allow us to have all the clinical information necessary for a good analysis of the patient's occlusion.

Likewise and advantageously in our invention, the same principle of the intervention of time in following the movements will be applied for measuring the pressure on the pathologies that can be found in the mouth. Indeed, we know that a pathology can i.a. be identified by its reaction to the pressure (more or less rapid return to its original position). By following the "physical" reaction over time of the optical impression of our excrescence, we will be able to assist in diagnosing. In fact, we took care, as can be seen in drawing 6a (69) to permit the passing-through of an instrument to perform this action, without it being an obligation of course.

The light is intended only to illuminate the scene, in order to promote the signal-noise ratio. It would indeed be possible to perform a measurement without light illuminating the surface being measured, but working in dark areas like the inside of the mouth requires an ambient light chosen as close as possible to daylight, or using a light having known spectral characteristics, so that the color rendering can be analyzed for extracting from same the characteristic data of the analyzed tissues.

This unstructured light also permits, as we already said, to work with the lighting of the dentist's room or the laboratory. Likewise, as we can see, by selecting certain wavelengths emitted by the LEDs present around the reading window and by increasing their frequencies or/and their intensities, we can place on a 3D image the display of certain anatomies or pathologies located at a small depth. Knowledge of the volume provides an indication of the positioning of this pathological limit, which permits to predict and display its evolution. This is also true for the fluorescence reactions of some tissues to blue or UV radiation. The fluorescence appears not only at the surface, but also in the depth of the pathology, which helps us to provide assistance for the therapy to be applied (exeresis of pathological tissue). Knowing the penetration of such or such radiation, it is possible to assess the extent and depth with respect to the actual 3D surface being analyzed.

Finally, and this is not restrictive, having two 2D images for constructing the 3D image permits us, in real time, to switch our vision without any modification of the camera to 2D color displays like all the cameras nowadays available on the market of dentistry. Therefore, since it does not use structured-light projection, our camera can perform all presently known functions, including zoom effects, but also the applications of color diagnosis on 2D images, such as the detections of caries by fluorescence in green, blue or UV (500 to 300 nm) radiations or visualizations in red and IR radiation (600 to 900 nm), depending on the LEDs that we have emulated in the analysis.

Advantageously, and this remains a very interesting point of our invention, it is possible to work in 2D color starting from 3D views. This can be done in two different ways;

- Since we use daylight (79), without projection of frames or other structured light, the display screen (5) in our control during the recording of pictures (78) allows us to use this optical impression camera as a simple 2D camera, which significantly limits the practitioners' cost of investment.
- We can also perform this 2D display, after digital processing and highlighting of the pathological areas by scanning with LEDs of specific wavelengths. This technique is obviously possible only starting from 3D images.

This same zoom effect in color picture or the emulations can be performed on the 3D images. It is obvious that the transition from color to grayscale will only be an offset function present in the software controlling the processing of images resulting from the operation of the camera.

It clearly appears from the foregoing description that the present invention fully solves the the problems set forth, in that it provides a real answer for optimizing 3D color and dynamic dental reading (in time) and the pathological analysis of skin pathologies at particularly low cost due to a concept that can be fixed during the manufacturing phase. It also clearly appears from this description that it permits to solve the basic problems, such as the control of the clinical procedure, especially since no alternative has been provided.

## EP 2 729 048 B1

**Claims**

1. Three-dimensional measuring device used in the dental field and aimed at measuring in the absence of projection of active or structured light, comprising means for capturing images as well as data-processing means for said images, said image-capturing means (38, 39) consist of means designed capable of permitting to capture simultaneously, or almost simultaneously, at least two images, a first and a second image, wherein said first image is totally or partially included in the said second image, said first image describing a narrower field than that of said second image, and having a higher accuracy than that said second image, **characterized in that** the device further comprises means for projecting at least one circle of colored light surrounding the field of said first image, and/or the field of said second image, to facilitate the reading in the mouth by the practitioner.

2. Three-dimensional measuring device according to claim 1, wherein the image-capturing means consist of at least two electronic image sensors, one of which (38) viewing a wide field with average accuracy and the other one (39) a narrower field with higher accuracy totally or partially included in said wide field, said sensors being associated with optical systems.

3. Three-dimensional measuring device according to claim 2, wherein the optical systems associated with the sensors have different focal lengths in order to permit two different levels of accuracy.

4. Three-dimensional measuring device according to claim 3, wherein the sensors consist of color or monochromatic CCD or CMOS electronic sensors.

5. Three-dimensional measuring device according to any of claims 1 to 4, wherein it comprises in addition an accelerometer/gyro/3D magnetometer (52) capable of providing a general and continuous information on the spatial position of the image-capturing means.

6. Three-dimensional measuring device according to any of claims 1 to 5, wherein it comprises: a central management and analog/digital data conversion unit,

    - a data transmission via cable, telephone or wireless,
    - a hardware system for additional processing, dialog/ display with the operator, data transmission and storage,
    - a power-supply card capable of operating on USB or on battery (e.g. AC/DC).

7. Three-dimensional measuring device according to any of claims 1 to 6, wherein it comprises a passive and unstructured lighting by means of LEDs of one or more wavelengths permitting to measure specular or Lambertian regular surfaces, and having unstructured light, but with the specific characteristics in terms of purity (consistent or not), type (color) and intensity (power) for the function of diagnosis on a 3D image, transferred onto the 3D surfaces.

8. Three-dimensional measuring device according to claim 7, wherein the LEDs are of a predefined wavelength.

9. Three-dimensional measuring device according to any of claims 2 to 8, wherein one of the sensors is designed capable of indicating the general information on the field depth, so that the focal length of the other sensor is pre-positioned in a region close to reality analyzed by the first sensor.

10. Three-dimensional measuring device according to any of claims 1 to 9, wherein the means for capturing images in the narrowest field with higher accuracy is associated with a displacement means permitting it to quickly scan the entire field covered by the other capturing means.

11. Three-dimensional measuring device according to any of claims 1 to 10, wherein the means for capturing images in the narrowest field with higher accuracy is associated with a variable zoom.

12. Three-dimensional measuring device according to any of claims 1 to 11, wherein it comprises a flash system with very fast pulsing LEDs.

13. Three-dimensional measuring device according to any of claims 1 to 12, wherein the optical systems include liquid-type lenses.

14. Three-dimensional measuring device according to any of claims 1 to 12, wherein the optical systems comprise lenses of glass or molded glass/plastic with a pupil on the input face, associated with a micromotor for adjusting the field depth.

15. Three-dimensional measuring device according to any of claims 1 to 12, wherein the optical systems comprise so-called "free-form" thermoplastic lenses comprised of a flat top surrounded by asymmetric facets.

**Patentansprüche**

1. Dreidimensionale Messvorrichtung, die auf dem Dentalgebiet verwendet wird und auf ein Messen, in Abwesenheit einer Projektion, von aktivem oder strukturiertem Licht zielt, umfassend Mittel zum Aufnehmen von Bildern sowie Datenverarbeitungsmittel für die Bilder, wobei die Bildaufnahmemittel (38, 39) aus Mitteln bestehen, die ausgestaltet sind, um dazu in der Lage zu sein, es gleichzeitig oder nahezu gleichzeitig zu ermöglichen mindestens zwei Bilder, ein erstes und ein zweites Bild, aufzunehmen, wobei das erste Bild vollständig oder teilweise in dem zweiten Bild eingeschlossen ist, wobei das erste Bild ein schmaleres Feld als das des zweiten Bildes beschreibt und eine höhere Genauigkeit als das zweite Bild aufweist, **dadurch gekennzeichnet, dass** die Vorrichtung ferner Mittel zum Projizieren mindestens eines Kreises von farbigem Licht umfasst, der das Feld des ersten Bildes umgibt, und/oder das Feld des zweiten Bildes, um das Lesen im Mund durch den Arzt zu erleichtern.

2. Dreidimensionale Messvorrichtung nach Anspruch 1, wobei die Bildaufnahmemittel aus mindestens zwei elektronischen Bildsensoren bestehen, von denen (38) eines ein breites Feld mit einer durchschnittlichen Genauigkeit und das andere (39) ein schmaleres Feld mit einer höheren Genauigkeit, das vollständig oder teilweise in dem breiten Feld eingeschlossen ist, ansieht, wobei die Sensoren optischen Systemen zugeordnet sind.

3. Dreidimensionale Messvorrichtung nach Anspruch 2, wobei die den Sensoren zugeordneten optischen Systeme unterschiedliche Brennweiten aufweisen, um zwei unterschiedliche Genauigkeitsniveaus zu ermöglichen.

4. Dreidimensionale Messvorrichtung nach Anspruch 3, wobei die Sensoren aus elektronischen Farb- oder Schwarz-Weiß-Sensoren, CCD-Sensoren oder CMOS-Sensoren bestehen.

5. Dreidimensionale Messvorrichtung nach einem der Ansprüche 1 bis 4, wobei diese zusätzlich einen/ein Beschleunigungsmesser/Gyro-/3D-Magnetometer (52) umfasst, der/das in der Lage ist, allgemeine und kontinuierliche Informationen zu der räumlichen Position des Bildaufnahmemittel bereitzustellen.

6. Dreidimensionale Messvorrichtung nach einem der Ansprüche 1 bis 5, wobei diese umfasst: eine zentrale Verwaltungs- und eine Analog-/Digitaldaten-Umwandlungseinheit,

   - eine Datenübertragung über Kabel, Telefon oder drahtlos,
   - ein Hardware-System zur zusätzlichen Verarbeitung, Dialog/Anzeige mit dem Bediener, Datenübertragung und Speicherung,
   - eine Stromversorgungskarte, die in der Lage ist, auf USB oder auf Batterie (z. B. Wechselstrom/Gleichstrom) zu arbeiten.

7. Dreidimensionale Messvorrichtung nach einem der Ansprüche 1 bis 6, wobei diese eine passive und unstrukturierte Beleuchtung mittels LEDs einer oder mehrerer Wellenlängen umfasst, die es ermöglichen, spiegelnde oder Lambertsche regelmäßige Oberflächen zu messen, und unstrukturiertes Licht aufweisen, jedoch mit den spezifischen Eigenschaften in Bezug auf Reinheit (konsistent oder nicht), Typ (Farbe) und Intensität (Leistung) für die Funktion der Diagnose auf einem 3D-Bild, das auf die 3D-Oberflächen übertragen wird.

8. Dreidimensionale Messvorrichtung nach Anspruch 7, wobei die LEDs eine vordefinierte Wellenlänge aufweisen.

9. Dreidimensionale Messvorrichtung nach einem der Ansprüche 2 bis 8, wobei einer der Sensoren dazu ausgestaltet ist, um in der Lage zu sein, die allgemeinen Informationen über die Feldtiefe anzugeben, sodass die Brennweite des anderen Sensors in einem Bereich nahe der Realität, analysiert durch den ersten Sensor, vorpositioniert ist.

10. Dreidimensionale Messvorrichtung nach einem der Ansprüche 1 bis 9, wobei das Mittel zum Aufnehmen von Bildern in dem schmalsten Feld mit höherer Genauigkeit einem Verschiebemittel zugeordnet ist, das es ermöglicht, das gesamte Feld, das durch das andere Aufnahmemittel abgedeckt wird, schnell abzutasten.

**11.** Dreidimensionale Messvorrichtung nach einem der Ansprüche 1 bis 10, wobei das Mittel zum Aufnehmen von Bildern in dem schmalsten Feld mit höherer Genauigkeit einem variablen Zoom zugeordnet ist.

**12.** Dreidimensionale Messvorrichtung nach einem der Ansprüche 1 bis 11, wobei diese ein Blitzsystem mit sehr schnell pulsierenden LEDs umfasst.

**13.** Dreidimensionale Messvorrichtung nach einem der Ansprüche 1 bis 12, wobei die optischen Systeme flüssigkeitsartige Linsen einschließen.

**14.** Dreidimensionale Messvorrichtung nach einem der Ansprüche 1 bis 12, wobei die optischen Systeme Linsen aus Glas oder geformtem Glas/Kunststoff mit einer Pupille auf der Eingabefläche umfassen, die einem Mikromotor zum Einstellen der Feldtiefe zugeordnet ist.

**15.** Dreidimensionale Messvorrichtung nach einem der Ansprüche 1 bis 12, wobei die optischen Systeme sogenannte "frei geformte" thermoplastische Linsen umfassen, die aus einer flachen Oberseite gebildet sind, die von asymmetrischen Facetten umgeben ist.


**Revendications**

**1.** Dispositif de mesure en trois dimensions utilisé dans le domaine dentaire et conçu pour réaliser des mesures en l'absence de projection d'une lumière active ou structurée, comprenant des moyens de capture d'images ainsi que des moyens de traitement de données pour lesdites images, lesdits moyens de capture d'images (38, 39) consistant en des moyens conçus pour permettre de capturer simultanément, ou presque simultanément, au moins deux images, une première et une seconde image, ladite première image étant totalement ou partiellement incluse dans ladite seconde image, ladite première image décrivant un champ plus étroit que celui de ladite seconde image, et présentant une précision supérieure à celle de ladite seconde image, **caractérisé en ce que** le dispositif comprend en outre des moyens pour projeter au moins un cercle de lumière colorée entourant le champ de ladite première image, et/ou le champ de ladite seconde image, afin de faciliter la lecture dans la bouche du praticien.

**2.** Dispositif de mesure en trois dimensions selon la revendication 1, dans lequel les moyens de capture d'images sont constitués d'au moins deux capteurs d'images électroniques, dont l'un (38) visualise un champ large avec une précision moyenne et l'autre (39) un champ plus étroit avec une précision supérieure totalement ou partiellement incluse dans ledit champ large, lesdits capteurs étant associés à des systèmes optiques.

**3.** Dispositif de mesure en trois dimensions selon la revendication 2, dans lequel les systèmes optiques associés aux capteurs ont des longueurs focales différentes afin de permettre deux niveaux de précision différents.

**4.** Dispositif de mesure en trois dimensions selon la revendication 3, dans lequel les capteurs sont des capteurs électroniques CCD ou CMOS de couleur ou monochromatiques.

**5.** Dispositif de mesure en trois dimensions selon l'une quelconque des revendications 1 à 4, **caractérisé en ce qu'**il comprend en outre un magnétomètre accéléromètre/gyro/3D (52) capable de fournir une information générale et continue sur la position spatiale des moyens de capture d'images.

**6.** Dispositif de mesure en trois dimensions selon l'une quelconque des revendications 1 à 5, dans lequel il comprend : une unité centrale de gestion et de conversion analogique/numérique de données,

- une transmission de données via câble, téléphone ou sans fil,
- un système matériel pour traitement supplémentaire, dialogue/affichage avec l'opérateur, transmission et stockage de données,
- une carte d'alimentation capable de fonctionner sur USB ou sur batterie (par ex. CA/CC).

**7.** Dispositif de mesure en trois dimensions selon l'une quelconque des revendications 1 à 6, dans lequel il comprend un éclairage passif et non structuré au moyen de LED d'une ou plusieurs longueurs d'onde permettant de mesurer des surfaces régulières spéculaire ou lambertienne, et ayant une lumière non structurée, mais présentant les caractéristiques spécifiques en termes de pureté (cohérente ou non), de type (couleur) et d'intensité (puissance) pour la fonction de diagnostic sur une image 3D, transférée sur les surfaces 3D.

**8.** Dispositif de mesure en trois dimensions selon la revendication 7, dans lequel les LED ont une longueur d'onde prédéfinie.

**9.** Dispositif de mesure en trois dimensions selon l'une quelconque des revendications 2 à 8, dans lequel l'un des capteurs est conçu pour indiquer les informations générales sur la profondeur de champ, de sorte que la longueur focale de l'autre capteur est pré-positionnée dans une région proche de la réalité analysée par le premier capteur.

**10.** Dispositif de mesure en trois dimensions selon l'une quelconque des revendications 1 à 9, dans lequel les moyens de capture d'images dans le champ le plus étroit de précision supérieure sont associés à un moyen de déplacement leur permettant d'analyser rapidement l'ensemble du champ couvert par les autres moyens de capture.

**11.** Dispositif de mesure en trois dimensions selon l'une quelconque des revendications 1 à 10, dans lequel les moyens de capture d'images dans le champ le plus étroit de précision supérieure sont associés à un zoom variable.

**12.** Dispositif de mesure en trois dimensions selon l'une quelconque des revendications 1 à 11, dans lequel il comprend un système de flash avec des LED à impulsions très rapides.

**13.** Dispositif de mesure en trois dimensions selon l'une quelconque des revendications 1 à 12, dans lequel les systèmes optiques comportent des lentilles de type liquide.

**14.** Dispositif de mesure en trois dimensions selon l'une quelconque des revendications 1 à 12, dans lequel les systèmes optiques comprennent des lentilles de verre ou de verre moulé/plastique avec une pupille sur la face d'entrée, associées à un micro-moteur pour ajuster la profondeur de champ.

**15.** Dispositif de mesure en trois dimensions selon l'une quelconque des revendications 1 à 12, dans lequel les systèmes optiques comprennent des lentilles thermoplastiques dites « libres » constituées d'un dessus plat entouré de facettes asymétriques.

EP 2 729 048 B1

Figure 1a

Figure 1b

29

1 to 2 cm

1 to 2 cm

2 to 4 cm

20 to 25 cm

Figure 2

14    10   7    18    11   8      9      12    13

17   15    16

Figure 3

Figure 4

Figure 5

10 to 30 mm.

2 mm

20 mm

10 mm

0,5 mm.

15 mm

30 mm

Figure 6

Figure 7

Figure 8a

Figure 8b

Figure 8c

Figure 9a

Figure 9b

Figure 10a

Figure 10b

Figure 10c

Figure 11

## General diagram

Time 1: press the button or pedal 1

**Real-time**

Acquisition phase

53

| Recording of macro-picture | Recording of micro-picture | Activation of the LEDs | Scanning and following times = Recording of additional pictures |

3D reconstruction *(local/regional/with and without accelerometer)*
Verification/validation

Display/User feedback on precision

54    55

**Off-line**

56 — Scaling

57 — Dense reconstruction with improved precision

Information about the patient

Setup of the files for storing and transmission

Transmission of the data internet Wifi

Figure 12

# Strategy of integration of recording of micro- and macro-pictures
# CASE 1 : 2 cameras, no accelerometer

Figure 13a

**Strategy of integration of recording of micro- and macro-picture**
**CASE 2 : with accelerometer and in regional reconstruction**

Figure 13b

Figure 13b

Strategy of integration of recording of micro- and macro-picture
CASE 3 : with accelerometer and in regional reconstruction

Figure 13c

Regional 3D reconstruction

Figure 14

64  63

Figure 15

65

Figure 16

66

67

Figure 17

```
┌──────────────────┐                    ┌──────────────────┐
│  Optical traces  │                    │    Projection    │
│                  │                    │     matrices     │
└──────────────────┘                    └──────────────────┘
          ╲                                      ╱
           ╲                                    ╱
            ╲                                  ╱
             ▼                                ▼
           ┌──────────────────────────────┐
    68     │        Assessment            │
           │      of the global           │
           │        geometry              │
           └──────────────────────────────┘
                        │
                        ▼
           ┌──────────────────────────────┐
    69     │        Space carving         │
           └──────────────────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 8010967
- US 4663720 A
- US 4742464 A
- BE 0091876
- US 4611288 A
- EP 0110797 A
- US 5092022 A
- FR 8405173
- FR 8702339
- US 4952149 A
- FR 9208128
- WO 9400074 A
- FR 8206707
- FR 8220349
- WO 4575805 A, Moermann and Brandestini
- WO 4837732 A
- US 6409504 B, Jones, T.N.
- US 0109559 A
- US 20100303341 A, G. Hausler
- US 20090227875 A, Denzen Cao
- EP 2166303 A, Steinbichler Opt.
- US 7372642 B, Rohaly and Co.
- US 2008038688 A1
- US 2010106275 A1
- EP 1756771 A
- EP 0600128 A
- EP 0373077 A
- EP 939131736 A
- EP 0369908 A
- US 5143086 A

### Non-patent literature cited in the description

- **G HAUSLER ; COL.** light sectioning with large depth and high resolution. *Appl. Opt.,* 1988, vol. 27
- **M. ALTSCHULER ; COL.** Numerical stereo camera. *SPIE,* 1981, vol. 283 3-D
- **M HALIOUA ; COL.** Automated phase measuring profilometry of 3D diffuse objects. *Appl.Opt.,* 1984, vol. 23
- **F.DURET.** the great adventure of CADCAM at IDS in Cologne. *dental floss No. 63,* May 2011, 14-26
- *Die Cerec Computer Reconstruction,* 1989
- *CAD/CIM in Aesthetic Dentistry,* 1996
- *State of the art of CAD/CAM restoration,* 2006
- **D. REKOW.** *J. of Dent.Practice Administration,* 1984, vol. 4 (2), 52-55
- **C. TOMASI ; COL.** Shape and motion from image streams under Orthography ; a factorization Method. *Int. J. of Computer Vision,* 1992, vol. 9 (2
- **BEUTTELL, J.** *Int.J.Cumputerized Dent.,* 1998, vol. 1, 35-39